(19) Europäisches Patentamt
European Patent Office
Office européen des brevets

(11) Veröffentlichungsnummer: **0 641 779 A1**

# (12) EUROPÄISCHE PATENTANMELDUNG

(21) Anmeldenummer: **94113488.4**

(22) Anmeldetag: **30.08.94**

(51) Int. Cl.⁶: **C07D 211/26**, C07D 265/30, A61K 31/445, A61K 31/535, C07D 405/12, C07D 401/12, C07D 401/14

(30) Priorität: **07.09.93 CH 2667/93**
**05.07.94 CH 2150/94**

(43) Veröffentlichungstag der Anmeldung:
**08.03.95 Patentblatt 95/10**

(84) Benannte Vertragsstaaten:
**AT BE CH DE DK ES FR GB GR IE IT LI LU MC NL PT SE**

(71) Anmelder: **F. HOFFMANN-LA ROCHE AG**
**Postfach 3255**
**CH-4002 Basel (CH)**

(72) Erfinder: **Ackermann, Jean**
**167 Elsässerstrasse**
**CH-4056 Basel (CH)**
Erfinder: **Banner, David**
**129 Neubadstrasse**
**CH-4054 Basel (CH)**
Erfinder: **Gubernator, Klaus**
**5 Carl-Maria-von-Weber-Strasse**
**D-79104 Freiburg (DE)**
Erfinder: **Hilpert, Kurt**
**5 Eichenstrasse**
**CH-4114 Hofstetten (CH)**
Erfinder: **Schmid, Gérard**
**Leibern**
**CH-4468 Kienberg (CH)**

(74) Vertreter: **Mahé, Jean et al**
**Postfach 3255**
**Grenzacherstrasse 124**
**CH-4002 Basel (CH)**

## (54) Neue 1-Amidinopiperidine und 4-Amidinomorphozine als Aggregationshemmer.

(57) Die neuen Carboxamide der Formel

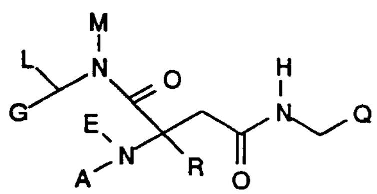

I

worin A,E,G,L,M, R und Q die in der Beschreibung angegebene Bedeutung haben, sowie Hydrate oder Solvate davon hemmen die durch Thrombin induzierte Plättchenaggregation und Gerinnung von Fibrinogen im Plasma. Man kann sie ausgehend von der entsprechenden Säure und dem entsprechenden Amin $H_2NCH_2Q$ herstellen.

EP 0 641 779 A1

Die Erfindung betrifft neue Carboxamide der Formel

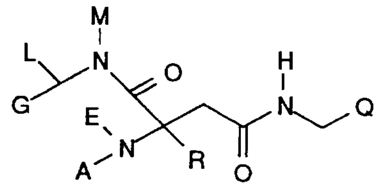

I

worin

| | |
|---|---|
| E | Wasserstoff, |
| G | H, nieder-Alkyl oder gegebenenfalls über nieder-Alkylen gebundenes COOH, COO-nieder-Alkyl, nieder-Alkanoyl, OH, nieder-Alkanoyloxy, nieder-Alkoxy, Aryl-niederalkoxy, $CONH_2$, $CONHCH_2CH_2OH$, CONHOH, $CONHOCH_3$, CONHO-Benzyl, $CONHSO_2$-nieder-Alkyl, $CONHCH_2CH_2$-Aryl, CONH-Cycloalkyl, $CONHCH_2$-Heteroaryl, $NH_2$, NHCOO-nieder-Alkyl, NHCOO-nieder-Aralkyl, $NHSO_3H$, ($NHSO_2$ oder $NHSO_3$)-nieder-Alkyl, NH-nieder-Alkanoyl, NHCOCOOH, NHCOCOO-nieder-Alkyl, NH-Cycloalkyl, NH-(3,4-Dioxo-2-hydroxy-cyclobut-1-enyl), NH-[2-nieder-(Alkoxy oder -Alkenyloxy)-3,4-dioxocyclobut-1-enyl], $NHCH_2$-Heteroaryl, NHCOCO-(Aryl oder nieder-Alkyl), $NHCOCH_2Cl$, NHCO-nieder-Alkylen-O-(niederalkyl oder aryl), $NHCOCH_2$[Aryl, Heteroaryl oder -N(Het)], NHCOC(NOH)-nieder-Alkylen-COOH, $NHSO_2$-N(Het), |
| $R^{10}$ und $R^{20}$ | H, nieder-Alkyl oder Phenyl, und |
| M | H, nieder-Alkyl oder -Alkenyl, Aryl, Heteroaryl, Cycloalkyl oder (Aryl, Heteroaryl oder Cycloalkyl)-niederalkyl, |
| L | H, nieder-Alkyl, Aryl, Cycloalkyl, oder (Aryl oder Cycloalkyl)-niederalkyl, oder |
| M und L | zusammen mit den Atomen, an die sie gebunden sind, eine Gruppe -N(Het) bilden, oder |
| E und G | zusammen eine Methylen- oder Carbonylgruppe bilden, und |
| M | H, nieder-Alkyl oder -Alkenyl, Aryl, Heteroaryl, Cycloalkyl oder (Aryl, Heteroaryl oder Cycloalkyl)-niederalkyl, oder Carboxy-niederalkyl, und |
| L | H, nieder-Alkyl, Aryl, Cycloalkyl, oder (Aryl oder Cycloalkyl)-niederalkyl, |
| A | H, Alkyl, nieder-Aralkyl oder eine Gruppe der Formel: |
| | - C(O)$R^2$ ($A^1$), |
| | -$S(O)_2N(R^3,R^4)$ ($A^2$) |
| | oder, falls die Gruppe Q eine Hydroxygruppe enthält, und/oder falls E und G zusammen $CH_2$ oder CO sind, dann |
| A | auch eine Gruppe der Formel: |
| | -$S(O)_2R^5$ ($A^3$) |
| | sein kann, |
| $R^2$ | nieder-Alkyl, gegebenenfalls über nieder-Alkylen gebundenes Aryl, Heteroaryl oder Cycloalkyl, oder über nieder-Alkylen gebundenes Carbo-niederalkoxy oder über nieder-Alkylen gebundenes -(O oder S)-(Aryl, Heteroaryl oder Cycloalkyl), wobei eine in $R^2$ enthaltene niederAlkylengruppe in $\alpha$-Stellung zur Carbonylgruppe, an der $R^2$ gebunden ist, durch Hydroxy, Amino oder nieder-Alkanoylamino substituiert sein kann, oder |
| $R^2$ | über nieder-Alkylen gebundenes Halogen, Carboxy, nieder-Alkoxy, Amino, Mono- oder Di-niederalkyl-amino oder eine über nieder-Alkylen gebundene Gruppe -N-(Het) oder |
| $R^2$ | eine Gruppe -$OR^{22}$ oder -$NHR^{22}$, |
| $R^{22}$ | nieder-Alkyl oder gegebenenfalls über nieder-Alkylen gebundenes Aryl, Heteroaryl oder Cycloalkyl, oder im nieder-Alkylanteil durch Aryl, Carbo-niederalkoxy |

| | |
|---|---|
| | oder COOH substituiertes nieder-Aralkyl, |
| $R^3$ und $R^4$ | unabhängig voneinander Wasserstoff, Alkyl oder Aryl-niederalkyl, oder zusammen mit dem N-Atom, an dem sie gebunden sind, eine Gruppe -N(Het) bilden, |
| $R^5$ | Aryl, Heteroaryl, Heterocyclyl, Alkyl oder Cycloalkyl, |
| -N(Het) | N-gebundenes, gegebenenfalls durch O, S, NH oder N-nieder-Alkyl unterbrochenes und gegebenenfalls durch bis zu 2 Substituenten aus der Gruppe von nieder-Alkyl, OH, Oxo, COOH, COO-nieder-Alkyl, $CH_2OH$ und $CH_2O$-Benzyl substituiertes $-N(CH_2)_{4-9}$, |
| Q | eine Gruppe der Formel $Q^1$ oder $Q^2$: |

($Q^1$) 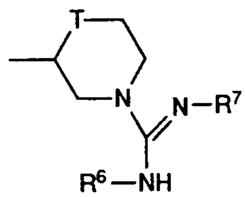 und ($Q^2$) 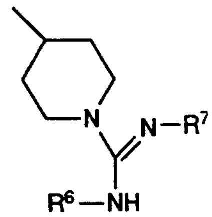

| | |
|---|---|
| T | $CH_2$ oder O, |
| eins von $R^6$ und $R^7$ | Wasserstoff oder Carbo-niederalkoxy und das andere Wasserstoff, Carbo-niederalkoxy oder Hydroxy, und |
| R | Wasserstoff oder nieder-Alkyl ist, |

sowie Hydrate oder Solvate und physiologisch verträgliche Salze davon.

Ferner betrifft die Erfindung Verfahren zur Herstellung der obigen Verbindungen, pharmazeutische Präparate, die solche Verbindungen enthalten, sowie die Verwendung dieser Verbindungen bei der Herstellung von pharmazeutischen Präparaten.

Beispiele von physiologisch verwendbaren Salzen der Verbindungen der Formel I sind Salze mit physiologisch verträglichen Mineralsäuren, wie Salzsäure, Schwefelsäure, schweflige Säure oder Phosphorsäure; oder mit organischen Säuren, wie Methansulfonsäure, p-Toluolsulfonsäure, Essigsäure, Trifluoressigsäure, Zitronensäure, Fumarsäure, Maleinsäure, Weinsäure, Bernsteinsäure oder Salicylsäure. Die Verbindungen der Formel I mit sauren Gruppen, wie die Carboxygruppe, können auch Salze mit physiologisch verträglichen Basen bilden. Beispiele solcher Salze sind Alkalimetall-, Erdalkalimetall-, Ammonium- und Alkylammoniumsalze, wie das Na-, K-, Ca- oder Tetramethylammoniumsalz. Die Verbindungen der Formel I können auch in Form von Zwitterionen vorliegen.

Die Verbindungen der Formel I können solvatisiert, insbesondere hydratisiert sein. Die Hydratisierung kann im Zuge des Herstellungsverfahrens erfolgen oder allmählich als Folge hygroskopischer Eigenschalten einer zunächst wasserfreien Verbindung der Formel I auftreten.

Die Verbindungen der Formel I enthalten zumindest zwei asymmetrische C-Atome und können daher als Diastereomerengemisch oder als optisch reine Verbindung vorliegen.

Im Rahmen der Erfindung bezeichnet der Ausdruck "nieder" Gruppen, die 1 bis 6, vorzugsweise 1 bis 4 C-Atome enthalten. So bezeichnet nieder-Alkyl allein oder in Kombination geradkettige oder verzweigte, 1 bis 6, vorzugsweise 1 bis 4 C-Atome enthaltende Gruppen, wie Methyl, Aethyl, Propyl, Isopropyl, Butyl, Isobutyl, t-Butyl, 2-Butyl und Pentyl. Als Alkylgruppen sind die nieder-Alkylgruppen bevorzugt. Ein Beispiel für nieder-Alkylen ist Methylen.

Aryl bezeichnet Gruppen, wie Phenyl und 1- oder 2-Naphthyl, gegebenenfalls mit einem oder mehreren Substituenton, wie Halogen, z.B. Chlor, oder nieder-Alkyl oder Alkoxy, z.B. $CH_3$, t-Butyl, OH, $OCH_3$, Phenyl, $CF_3$, $OCF_3$, Cyclopentyl, CN, COOH, $COOCH_3$, $COOC_2H_5$, $CONH_2$ oder Tetrazolyl.

Heteroarylgruppen sind 5- bis 10-gliedrige aromatische Gruppen, die aus einem oder 2 Ringen bestehen und ein oder mehrere N- und/oder O-Atom(e) enthalten. Beispiele davon sind 2-, 3- oder 4-Pyridyl auch in Form ihrer N-Oxyde, Furyl, Pyrimidyl, Indolyl, Pyrazinyl, Pyridazinyl, Tetrazolyl, Oxadiazolyl, Chinolyl oder Imidazolyl. Sie können substituiert sein, z.B. durch Oxo, nieder-Alkyl, wie $CH_3$, Halogen, wie Chlor, oder Amino.

Cycloalkylgruppen enthalten 3 bis 8 C-Atome. Beispiele davon sind Cyclopropyl, Cyclopentyl und Cyclohexyl.

Heterocyclyl bezeichnet 5- bis 10-gliedrige nicht aromatische, teilweise oder vollständig gesättigte Gruppen, wie Tetrahydrochinolyl oder Tetrahydropyridazinyl, die ein oder zwei Ringe und zumindest ein Heteroatom, z.B. ein oder zwei N-Atome, enthalten und gegebenenfalls durch einem oder mehreren

Substituenten, wie nieder-Alkyl, z. B. Methyl, substituiert sind.

Beispiele von gegebenenfalls durch einem Heteroatom unterbrochenen und gegebenenfalls substituierten Tetra- bis Nonamethyleniminogruppen N(Het) sind Hexahydroazepin, Morpholino und Methylpiperazinyl.

Beispiele von erfindungsgemässen Verbindungen sind diejenigen der Formel

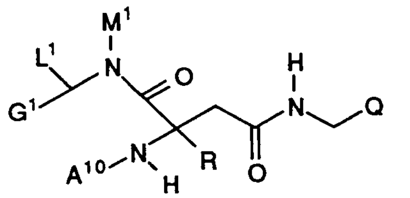

Ia

worin

| | |
|---|---|
| $G^1$ | H, nieder-Alkyl oder gegebenenfalls über nieder-Alkylen gebundenes COOH, COO-nieder-Alkyl, nieder-Alkanoyl, OH, nieder-Alkanoyloxy, nieder-Alkoxy, Arylniederalkoxy, $CONH_2$, $CONHCH_2CH_2OH$, CONHOH, $CONHOCH_3$, CONHO-Benzyl, $CONHSO_2$-nieder-Alkyl, $CONHCH_2CH_2$-Aryl, CONH-Cycloalkyl, $CONHCH_2$-Heteroaryl, $NH_2$, NHCOO-nieder-Alkyl, NHCOO-nieder-Aralkyl, $NHSO_3H$, ($NHSO_2$ oder $NHSO_3$)-nieder-Alkyl, NH-nieder-Alkanoyl, NHCOCOOH, NHCOCOO-nieder-Alkyl, NH-Cycloalkyl, NH-(3,4-Dioxo-2-hydroxy-cyclobut-1-enyl), NH-[2-nieder-(Alkoxy oder -Alkenyloxy)-3,4-dioxocyclobut-1-enyl], $NHCH_2$-Heteroaryl, NHCOCO-(Aryl oder nieder-Alkyl), $NHCOCH_2Cl$, NHCO-nieder-Alkylen-O-(niederalkyl oder aryl), $NHCOCH_2$[Aryl, Heteroaryl oder -N(Het)], NHCOC-(NOH)-nieder-Alkylen-COOH, $NHSO_2$-N(Het), NHCO-(Aryl, Heteroaryl oder Heterocyclyl), $NHPO_3(R^{10},R^{20})$, Heteroaryl oder eine Gruppe CO-N(Het), |
| $R^{10}$ und $R^{20}$ | H, nieder-Alkyl oder Phenyl, und |
| $M^1$ | H, nieder-Alkyl oder -Alkenyl, Aryl, Heteroaryl, Cycloalkyl oder (Aryl, Heteroaryl oder Cycloalkyl)-niederalkyl, |
| $L^1$ | H, nieder-Alkyl, Aryl, Cycloalkyl, oder (Aryl oder Cycloalkyl)-niederalkyl, oder |
| $M^1$ und $L^1$ | zusammen mit den Atomen, an die sie gebunden sind eine Gruppe -N(Het) bilden, |
| $A^{10}$ | H, Alkyl, nieder-Aralkyl oder eine Gruppe der Formel: |

$-C(O)R^2$ ($A^1$),

$-S(O)_2N(R^3,R^4)$ ($A^2$)

oder, falls die Gruppe Q eine Hydroxygruppe enthält, dann

$A^{10}$ auch eine Gruppe der Formel

$-SO_2(R^5)$ ($A^3$)

sein kann, und

R, $R^2$ bis $R^5$, -N(Het) und Q die obige Bedeutung haben.

Weitere Beispiele von erfindungsgemässen Verbindungen sind diejenigen der Formel

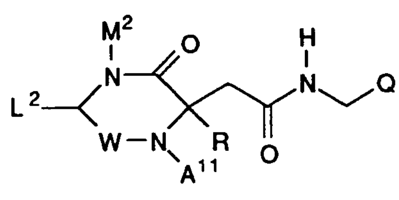

Ib

worin

W Methylen oder Carbonyl,

| | |
|---|---|
| $M^2$ | H, nieder-Alkyl oder -Alkenyl, Aryl, Heteroaryl, Cycloalkyl oder (Aryl, Heteroaryl oder Cycloalkyl)-niederalkyl, oder Carboxy-niederalkyl, und |
| $L^2$ | H, nieder-Alkyl, Aryl, Cycloalkyl, oder (Aryl oder Cycloalkyl)-niederalkyl, und |
| $A^{11}$ | H, Alkyl, nieder-Aralkyl oder eine Gruppe der Formel: |
| | $-C(O)R^2$ ($A^1$), |
| | $-S(O)_2N(R^3,R^4)$ ($A^2$) |
| | oder |
| | $-S(O)_2R^5$ ($A^3$) |
| | ist, und |
| R, $R^2$ bis $R^5$ und Q | die obige Bedeutung haben. |

Unter den Verbindungen Ia sind diejenigen bevorzugt, worin $L^1$ Wasserstoff und $G^1$ gegebenenfalls über nieder-Alkylen gebundenes COOH, COO-nieder-Alkyl, NHCOO-nieder-Aralkyl oder NHCO(Aryl oder Heteroaryl) ist,

ferner diejenigen, worin $M^1$ nieder-Alkyl oder Cycloalkyl ist und/oder worin $A^{10}$ eine Gruppe der Formel

$-C(O)-R^2$ ($A^1$)

in der $R^2$ eine Gruppe $R^{22}$, $-OR^{22}$ oder $-NHR^{22}$ ist, und $R^{22}$ nieder-Alkyl oder gegebenenfalls über nieder-Alkylen gebundenes Aryl, Heteroaryl oder Cycloalkyl ist, oder in der $R^2$ über nieder-Alkylen gebundenes Carbo-niederalkoxy oder über nieder-Alkylen gebundenes -(O oder S)-(Aryl oder Heteroaryl) ist, wobei eine in $R^2$ enthaltene nieder-Alkylengruppe in $\alpha$-Stellung zur Carbonylgruppe, an der $R^2$ gebunden ist, durch OH oder nieder-Alkanoylamino substituiert sein kann.

Weitere bevorzugte Verbindungen Ia sind diejenigen, worin $A^{10}$ eine Gruppe $-S(O)_2$ Aryl und Q eine Gruppe OH enthält, oder worin $A^{10}$ Morpholinosulfonyl ist.

Unter den Verbindungen Ia sind ferner diejenigen bevorzugt, worin $L^1$ Wasserstoff und $G^1$ gegebenenfalls über nieder-Alkylen gebundenes NHCO-nieder-Alkylen-O-(niederalkyl oder aryl), $NHCOCH_2$[Aryl, Heteroaryl oder -N(Het)], NHCOC(NOH)-nieder-Alkylen-COOH, $NHSO_2$-N(Het), NHCO-Heterocyclyl oder gegebenenfalls durch O oder S unterbrochenes und gegebenenfalls durch bis zu 2 Substituenten aus der Gruppe von nieder-Alkyl, COOH, COO-nieder-Alkyl, $CH_2OH$ und $CH_2O$-Benzyl substituiertes $CON(CH_2)_{4-9}$ ist,

und ferner diejenigen, worin $A^{10}$ H, nieder-Alkyl, nieder-Aralkyl oder eine Gruppe

$-C(O)-R^2$ ($A^1$)

in der $R^2$ über nieder-Alkylen gebundenes Halogen, Carboxy, nieder-Alkoxy, Amino, Mono- oder Diniederalkyl-amino oder eine über nieder-Alkylen gebundene Gruppe -N(Het) oder
$R^2$ eine Gruppe $-NHR^{22}$, worin $R^{22}$ eine im nieder-Alkylanteil durch Aryl, Carbo-niederalkoxy oder COOH substituierte nieder-Aralkylgruppe ist.

Beispiele von Verbindungen der Formel Ia sind folgende:

[[(S)-3-[(S)-1-(Amino-imino-methyl)-piperidin-3-ylmethylcarbamoyl]-2-benzyloxycarbonylamino-propionyl]-cyclopropyl-amino]-essigsäureethylester,

3-[[(S)-3-[(S)-1-(Amino-imino-methyl)-piperidin-3-ylmethylcarbamoyl]-2-benzyloxycarbonylamino-propionyl]-cyclopropyl-amino]-propionsäureethylester,

[[(S)-3-[(S)-1-(Amino-imino-methyl)-piperidin-3-ylmethylcarbamoyl]-2-benzyloxycarbonylamino-propionyl]-butyl-amino]-essigsäureethylester,

3-[[(S)-3-[(S)-1-(Amino-imino-methyl)-piperidin-3-ylmethylcarbamoyl]-2-benzyloxycarbonylamino-propionyl]-butyl-amino]-propionsäureethylester,

[[(S)-3-[(S)-1-(Amino-imino-methyl)-piperidin-3-ylmethylcarbamoyl]-2-benzyloxycarbonylamino-propionyl]-cyclopropyl-amino]-essigsäure,

3-[[(S)-3-[(S)-1-(Amino-imino-methyl)-piperidin-3-ylmethylcarbamoyl]-2-benzyloxycarbonylamino-propionyl]-cyclopropyl-amino]-propionsäure,

[[(S)-3-[(S)-1-(Amino-imino-methyl)-piperidin-3-ylmethylcarbamoyl]-2-benzyloxycarbonylamino-propionyl]-butyl-amino]-essigsäure,

3-[[(S)-3-[(S)-1-(Amino-imino-methyl)-piperidin-3-ylmethylcarbamoyl]-2-benzyloxycarbonylamino-propionyl]-butyl-amino]-propionsäure,

[[(S)-3-[4-(Amino-imino-methyl)-morpholin-2-ylmethylcarbamoyl]-2-benzyloxycarbonylamino-propionyl]-cyclopropyl-amino]-essigsäureethylester,

[[(S)-3-[4-(Amino-imino-methyl)-morpholin-2-ylmethylcarbamoyl]-2-benzyloxycarbonylamino-propionyl]-butyl-amino]-essigsäureethylester,

3-[[(S)-3-[4-(Amino-imino-methyl)-morpholin-2-ylmethylcarbamoyl]-2-benzyloxycarbonylamino-propionyl]-cyclopropyl-amino]-propionsäureethylester,

3-[[(S)-3-[4-(Amino-imino-methyl)-morpholin-2-ylmethylcarbamoyl]-2-benzyloxycarbonylamino-propionyl]-butyl-amino]-propionsäureethylester,

[[(S)-3-[4-(Amino-imino-methyl)-morpholin-2-ylmethylcarbamoyl]-2-benzyloxycarbonylamino-propionyl]-cyclopropyl-amino]-essigsäure,

3-[[(S)-3-[4-(Amino-imino-methyl)-morpholin-2-ylmethylcarbamoyl]-2-benzyloxycarbonylamino-propionyl]-cyclopropyl-amino]-propionsäure,

3-[[(S)-3-[4-(Amino-imino-methyl)-morpholin-2-ylmethylcarbamoyl]-2-benzyloxycarbonylamino-propionyl]-butyl-amino]-propionsäure,

[[(S)-3-[(S)-1-(Amino-imino-methyl)-piperidin-3-ylmethylcarbamoyl]-2-cyclohexylmethoxycarbonylamino-propionyl]-cyclopropyl-amino]-essigsäureethylester,

[[(S)-3-[(S)-1-(Amino-imino-methyl)-piperidin-3-ylmethylcarbamoyl]-2-cyclohexylmethoxycarbonylamino-propionyl]-cyclopropyl-amino]-essigsäure,

[[(S)-3-[(S)-1-(Amino-imino-methyl)-piperidin-3-ylmethylcarbamoyl]-2-(3-benzyl-ureido)-propionyl]-cyclopropyl-amino]-essigsäureethylester,

[[(S)-3-[(S)-1-(Amino-imino-methyl)-piperidin-3-ylmethylcarbamoyl]-2-(3-furan-2-ylmethyl-ureido)-propionyl]-cyclopropyl-amino]-essigsäureethylester,

[[(S)-3-[(S)-1-(Amino-imino-methyl)-piperidin-3-ylmethylcarbamoyl]-2-(3-butyl-ureido)-propionyl]-cyclopropyl-amino]-essigsäureethylester,

3-[[(S)-3-[(S)-1-(Amino-imino-methyl)-piperidin-3-ylmethylcarbamoyl]-2-(3-furan-2-ylmethyl-ureido)-propionyl]-butyl-amino]-propionsäureethylester,

[[(S)-3-[(S)-1-(Amino-imino-methyl)-piperidin-3-ylmethylcarbamoyl]-2-(3-benzyl-ureido)-propionyl]-cyclopropyl-amino]-essigsäure,

[[(S)-[(S)-1-(Amino-imino-methyl)-piperidin-3-ylmethylcarbamoyl]-2-(3-furan-2-ylmethyl-ureido)propionyl]-cyclopropyl-amino]-essigsäure,

[[(S)-3-[(S)-1-(Amino-imino-methyl)-piperidin-3-ylmethylcarbamoyl]-2-(3-n-butyl-ureido)-propionyl]-cyclopropyl-amino]-essigsäure,

[[(S)-[(S)-1-(Amino-imino-methyl)-piperidin-3-ylmethylcarbamoyl]-2-(3-furan-2-ylmethyl)-ureido)-propionyl]-n-butyl-amino]-propionsäure,

[[(S)-3-[(S)-1-(Amino-imino-methyl)-piperidin-3-ylmethylcarbamoyl]-2-pentanoylamino-propionyl]-cyclopropyl-amino]-essigsäureethylester,

[[(S)-3-[(S)-1-(Amino-imino-methyl)-piperidin-3-ylmethylcarbamoyl]-2-hexanoylamino-propionyl]-cyclopropyl-amino]-essigsäureethylester,

[[(S)-3-[(S)-1-(Amino-imino-methyl)-piperidin-3-ylmethylcarbamoyl]-2-(4-chloro-pyridin-2-ylcarbonylamino)-propionyl]-cyclopropyl-amino]-essigsäureethylester,

[[(S)-3-[(S)-1-(Amino-imino-methyl)-piperidin-3-ylmethylcarbamoyl]-2-benzoylamino-propionyl]-cyclopropyl-amino]-essigsäureethylester,

[[(S)-3-[(S)-1-(Amino-imino-methyl)-piperidin-3-ylmethylcarbamoyl]-2-benzyloxycarbonylamino-propionyl]-cyclopropyl-amino]-essigsäureethylester,

[[(S)-3-[(S)-1-(Amino-imino-methyl)-piperidin-3-ylmethylcarbamoyl]-2-hexanoylamino-propionyl]-cyclopropyl-amino]-essigsäure,

[[(S)-3-[(S)-1-(Amino-imino-methyl)-piperidin-3-ylmethylcarbamoyl]-2-(4-chloro-pyridin-2-ylcarbonylamino)-propionyl]-cyclopropyl-amino]-essigsäure,

[[(S)-3-[(S)-1-(Amino-imino-methyl)-piperidin-3-ylmethylcarbamoyl]-2-benzoylamino-propionyl]-cyclopropyl-amino]-essigsäure,

[[(S)-3-[(S)-1-(Amino-imino-methyl)-piperidin-3-ylmethylcarbamoyl]-2-benzylcarbonylamino-propionyl]-cyclopropyl-amino]-essigsäure,

(S)-N4-[(S)-1-(Amino-imino-methyl)-piperidin-3-ylmethyl]-2-tert-butoxycarbonylamino-N1-cyclopropyl-N1-(2-pyrazin-2-ylcarbonylamino)-succinamid,

(S)-N4-[(S)-1-(Amino-imino-methyl)-piperidin-3-ylmethyl]-N1-cyclopropyl-2-(3-indol-3-yl-propionylamino)-N1-(2-pyrazin-2-ylcarbonylaminoethyl)-succinamid,

(S)-4-[(S)-2-[(S)-1-(Amino-imino-methyl)-piperidin-3-ylmethylcarbonyl]-1-[cyclopropyl-(2-pyrazin-2-ylcarbonylamino-ethyl)-carbamoyl]-ethylcarba moyl]-4-hydroxy-buttersäuremethylester,

(S)-2-[(S)-2-Acetylamino-3-indol-3-ylpropionylamino]-N4-[(S)-1-(amino-imino-methyl)-piperidin-3-ylmethyl]-N1-cyclopropyl-N1-(2-pyrazin-2-ylcarbonylamino-ethyl)-succinamid,

(S)-N4-[(S)-1-(Amino-imino-methyl)-piperidin-3-ylmethyl]-N1-cyclopropyl-2-phenoxyacetylamino-N1-(2-pyrazin-2-ylcarbonylamino-ethyl)-succinamid,

2-[[(S)-3-[(S)-1-(Amino-imino-methyl)-piperidin-3-ylmethylcarbamoyl]-2-morpholin-4-ylsulfonylamino-propionyl]-cyclopropyl-amino]-ethylcarbaminsäurebenzylester und

(S)-N4-[(S)-1-(Amino-imino-methyl)-piperidin-3-ylmethyl]-N1-cyclopropyl-2-morpholin-4-ylsulfonylamino-N1-(2-pyrazin-2-ylcarbonylaminoethyl)-succinamid.

Weitere Beispiele von Verbindungen Ia sind:

(S)-N4-[(S)-1-(Amino-imino-methyl)-piperidin-3-ylmethyl]-N1-cyclopropyl-2-morpholin-4-ylsulfonylamino-N1-[2-(6-oxo-1,4,5,6-tetrahydro-pyridazin-3-ylcarbonylamino)-ethyl]-succinamid,

(S)-N4-[(S)-1-(Amino-imino-methyl)-piperidin-3-ylmethyl]-N1-[2-(3-amino-pyrazin-2-ylcarbonylamino)-ethyl]-N1-cyclopropyl-2-morpholin-4-ylsulfonylamino-succinamid,

(S)-N4-[(S)-1-(Amino-imino-methyl)-piperidin-3-ylmethyl]-N1-cyclopropyl-N1-(2-methoxyacetylamino-ethyl)-2-morpholin-4-ylsulfonylamino-succinamid,

(S)-N4-[(S)-1-(Amino-imino-methyl)-piperidin-3-ylmethyl]-N-1-cyclopropyl-N1-(2-morpholin-4-ylacetylamino-ethyl)-2-morpholin-4-ylsulfonyl-amino-succinamid,

(S)-N4-[(S)-1-(Amino-imino-methyl)-piperidin-3-ylmethyl]-N1-cyclopropyl-N1-[2-(4-methyl-piperazin-1-ylacetylamino)-ethyl]-2-morpholin-4-ylsulfonylamino-succinamid,

(S)-N4-[(S)-1-(Amino-imino-methyl)-piperidin-3-ylmethyl-N1-cyclopropyl-N1-[2-(3-methoxy-propionylamino)-ethyl]-2-morpholin-4-ylsulfonylamino-succinamid,

(S)-N4-[(S)-1-(Amino-imino-methyl)-piperidin-3-ylmethyl]-N1-cyclopropyl-N1-(2-imidazol-1-ylacetylamino-ethyl)-2-morpholin-4-ylsulfonylamino-succinamid,

(E)- und (Z)-4-[2-[[(S)-3-[(S)-1-(Amino-imino-methyl)-piperidin-3-ylmethylcarbamoyl]-2-morpholin-4-ylsulfonylamino-propionyl]-cyclopropyl amino]-ethylcarbamoyl]-4-hydroxyimino-buttersäure,

(S)-N1-(2-Amino-ethyl)-N4-[(S)-1-(amino-imino-methyl)-piperidin-3-ylmethyl]-N1-cyclopropyl-2-morpholin-4-ylsulfonylamino-succinamid,

(S)-N4-[(S)-1-(Amino-imino-methyl)-piperidin-3-ylmethyl]-N1-cyclopropyl-N1-[2-(4-methyl-piperazin-1-ylsulfonylamino)-ethyl]-2-morpholin-4-ylsulfonylamino-succinamid,

(S)-N4-[(S)-1-(Amino-imino-methyl)-piperidin-3-ylmethyl-N1-cyclopropyl-2-morpholin-4-ylsulfonylamino-N1-(2-morpholin-4-ylsulfonylaminoethyl)-succinamid,

(S)-N4-[(S)-1-(Amino-imino-methyl)-piperidin-3-ylmethyl]-N1-cyclopropyl-2-(3-methoxy-propionylamino)-N1-(2-pyrazin-2-ylcarbonylamino-ethyl)-succinamid,

(S)-N4-[(S)-1-(Amino-imino-methyl)-piperidin-3-ylmethyl]-N1-cyclopropyl-2-methoxyacetylamino-N1-(2-pyrazin-2-ylcarbonylamino-ethyl)-succinamid,

(S)-N4-[(S)-1-(Amino-imino-methyl)-piperidin-3-ylmethyl]-N1-cyclopropyl-2-(4-methyl-piperazin-1-ylacetylamino)-N1-(2-pyrazin-2-ylcarbonylaminoethyl)-succinamid,

e) (S)-N4-[(S)-1-(Amino-imino-methyl)-piperidin-3-ylmethyl]-2[(S)-2-amino-3-phenyl-propionylamino]-N1-cyclopropyl-N1-(2-pyrazin-2-ylcarbonylamino-ethyl)-succinamid,

(S)-N4-[(S)-1-(Amino-imino-methyl)-piperidin-3-ylmethyl-2-[(R)-2-amino-3-phenyl-propionylamino]-N1-cyclopropyl-N1-(2-pyrazin-2-ylcarbonylamino-ethyl)-succinamid,

(S)-N4-[(S)-1-(Amino-imino-methyl)-piperidin-3-ylmethyl]-N1-cyclopropyl-2-[2-(4-hydroxy-piperidin-1-yl)-acetylamino]-N1-(2-pyrazin-2-ylcarbonylamino-ethyl)-succinamid,

(S)-N4-[(S)-1-(Amino-imino-methyl)-piperidin-3-ylmethyl]-N1-cyclopropyl-2-(2-imidazol-1-yl-acetylamino)-N1-(2-pyrazin-2-ylcarbonylamino-ethyl)-succinamid,

(S)-N4-[(S)-1-(Amino-imino-methyl)-piperidin-3-ylmethyl]-N1-cyclopropyl-2-[2-(2-oxo-piperidin-1-yl)-acetylamino]-N1-(2-pyrazin-2-ylcarbonylamino-ethyl)-succinamid,

(S)-N4-[(S)-1-(Amino-imino-methyl)-piperidin-3-ylmethyl]-N1-cyclopropyl-2-(3-dimethylamino-propionylamino)-N1-(2-pyrazin-2-ylcarbonylamino-ethyl)-succinamid,

(S)-N4-[(S)-1-(Amino-imino-methyl)-piperidin-3-ylmethyl]-N1-cyclopropyl-2-(2-dimethylamino-acetylamino)-N1-(2-pyrazin-2-ylcarbonylamino-ethyl)-succinamid,

(S)-N4-[(S)-1-(Amino-imino-methyl)-piperidin-3-ylmethyl]-N1-cyclopropyl-2-(2,6-dimethyl-morpholin-4-ylacetylamino)-N1-(2-pyrazin-2-ylcarbonylamino-ethyl)-succinamid,

3-[[(S)-3-[(S)-1-(Amino-imino-methyl)-piperidin-3-ylmethylcarbomoyl]-2-butylamino-propionyl]-cyclopropyl-amino]-propionsäureethylester,

3-[[(S)-3-[(S)-1-(Amino-imino-methyl)-piperidin-3-ylmethylcarbamoyl]-2-butylamino-propionyl]-cyclopropyl-amino]-propionsäure,

[[(S)-2-Amino-3-[(S)-1-(amino-imino-methyl)-piperidin-3-ylmethylcarbamoyl]-propionyl]-cyclopropyl-amino]-essigsäure,

(S)-3-Amino-N-[(S)-1-(amino-imino-methyl)-piperidin-3-ylmethyl]-4-azepan-1-yl-4-oxo-butyramid,

(S)-N-[(S)-1-(Amino-imino-methyl)-piperidin-3-ylmethyl]-4-azepan-1-yl-3-benzyloxycarbonylamino-4-oxo-butyramid,

[[(S)-3-[(S)-1-(Amino-imino-methyl)-piperidin-3-ylmethylcarbamoyl]-2-(3-diphenylmethyl-ureido)-propionyl]-cyclopropyl-amino]-essigsäureethylester,

[[(S)-3-[(S)-1-(Amino-imino-methyl)-piperidin-3-ylmethylcarbamoyl]-2-(3-cyclohexylmethyl-ureido)-propionyl]-cyclopropyl-amino]-essigsäureethylester,

(S)-N-[(S)-1-(Amino-imino-methyl)-piperidin-3-ylmethyl]-4-azepan-1-yl-3-(3-benzyl-ureido)-4-oxo-butyramid,

(S)-2-[3-[(S)-1-[(S)-(Amino-imino-methyl)-piperidin-3-ylmethylcarbamoylmethyl]-2-azepan-1-yl-2-oxo-ethyl]-ureido]-3-phenylpropionsäure-methylester,

(R)-2-[3-[(S)-1-[(S)-(Amino-imino-methyl)-piperidin-3-ylmethylcarbamoylmethyl]-2-azepan-1-yl-2-oxo-ethyl]-ureido]-3-phenylpropionsäure-methylester,

[[(S)-3-[(S)-1-(Amino-imino-methyl)-piperidin-3-ylmethylcarbamoyl]-2-(3-diphenylmethyl-ureido)-propionyl]-cyclopropyl-amino]-essigsäure,

[[(S)-3-[(S)-1-(Amino-imino-methyl)-piperidin-3-ylmethylcarbamoyl]-2-(3-cyclohexylmethyl-ureido)-propionyl]-cyclopropyl-amino]-essigsäure,

(S)-2-[3-[(S)-1-[(S)-(Amino-imino-methyl)-piperidin-3-ylmethylcarbamoylmethyl]-2-azepan-1-yl-2-oxo-ethyl]-ureido]-3-phenylpropionsäure,

(R)-2-[3-[(S)-1-[(S)-(Amino-imino-methyl)-piperidin-3-ylmethylcarbamoylmethyl]-2-azepan-1-yl-2-oxo-ethyl]-ureido]-3-phenylpropionsäure,

[[(S)-2-Benzyloxycarbonylamino-3-[(S)-1-(ethoxycarbonylamino-imino-methyl)-piperidin-3-ylmethylcarbamoyl]-propionyl]-cyclopropyl-amino]-essigsäure,

[[(S)-2-Benzyloxycarbonylamino-3-[4-(ethoxycarbonylamino-imino-methyl)-morpholin-2-ylmethylcarbamoyl]-propionyl]-cyclopropyl-amino]-essigsäure,

[[(S)-3-[(S)-1-Amino-imino-methyl)-piperidin-3-ylmethylcarbamoyl]-2-(2-chlor-acetylamino)-propionyl]-cyclopropyl-amino]-essigsäureethylester,

[[(S)-3-[(S)-1-Amino-imino-methyl)-piperidin-3-ylmethylcarbamoyl]-2-(2-chlor-acetylamino)-propionyl]-cyclopropyl-amino]-essigsäure.

Beispiele von Verbindungen Ib sind:

N-[(S)-1-(Amino-imino-methyl)-piperidin-3-ylmethyl]-2[(S)-4-cyclopentyl-1-naphthalen-2-ylsulfonyl-3,6-dioxo-piperazin-2-yl]-acetamid,

[3-[(S)-1-(Amino-imino-methyl)-piperidin-3-ylmethylcarbamoylmethyl]-4-butylsulfonyl-2-oxo-piperazin-1-yl]essigsäure und

N-[(S)-1-(Amino-imino-methyl)-piperidin-3-ylmethyl]-2-[(S)-2,6-dioxo-4-butyl-piperazin-2-yl]-acetamid.

Weitere Beispiele von Verbindungen Ib sind:

N-[(S)-1-(Amino-imino-methyl)-piperidin-3-ylmethyl]-2-[(S)-4-cyclopropyl-3,6-dioxo-1-(3-phenyl-propyl)-piperazin-2-yl]-acetamid,

N-[(S)-1-(Amino-imino-methyl)-piperidin-3-ylmethyl]-2-[(S)-1-naphthalen-2-ylsulfonyl-3,6-dioxo-piperazin-2-yl]-acetamid,

N-[(S)-1-(Amino-imino-methyl)-piperidin-3-ylmethyl]-2-[(2S,5S)-5-hydroxymethyl-1-naphthalen-2-ylsulfonyl-3,6-dioxo-piperazin-2-yl]-acetamid,

(2S,5R)- und (2S,5S)-(5-Benzyl-3,6-dioxo-4-propyl-piperazin-2-yl)essigsäure (S)-1-(amino-imino-methyl)-piperidin-3-ylmethylamid,

(S)-[2-[(S)-1-(Amino-imino-methyl)-piperidin-3-ylmethylcarbamoyl-methyl]-4-cyclopropyl-3,6-dioxo-piperazin-1-yl]-essigsäure-ethylester,

[(S)-2-[(S)-1-(Amino-imino-methyl)-piperidin-3-ylmethylcarbamoyl-methyl]-4-cyclopropyl-3,6-dioxo-piperazin-1-yl]-essigsäure,

(R)- und (S)-(2,4-Dimethyl-1-naphthalen-2-ylsulfonyl-3-oxo-piperazin-2-yl)-essigsäure-(S)-1-(amino-imino-methyl)-piperidin-3-ylmethylamid.

Bevorzugte Verbindungen der Formel I sind die folgenden:

[[(S)-3-[4-(Amino-imino-methyl)-morpholin-2-ylmethylcarbamoyl]-2-benzyloxycarbonylamino-propionyl]-butyl-amino]-essigsäure,

[[(S)-3-[(S)-1-(Amino-imino-methyl)-piperidin-3-ylmethylcarbamoyl]-2-pentanoylamino-propionyl]-cyclopropyl-amino]-essigsäure,

(S)-N4-[(S)-1-(Amino-imino-methyl)-piperidin-3-ylmethyl]-N1-cyclopropyl-N1-(2-pyrazin-2-ylcarbonylamino-ethyl)-2-pyrimidin-2-ylsulfanylacetylamino-succinamid,

(S)-N4-[(S)-1-(Amino-hydroxyimino-methyl)-piperidin-3-ylmethyl]-N1-cyclopropyl-2-(naphthalen-2-ylsulfonylamino)-N1-[2-(pyrazin-2-ylcarbonyl-amino)-ethyl]-succinamid,

(S)-N4-[(S)-1-(Amino-imino-methyl)-piperidin-3-ylmethyl]-N1-cyclopropyl-2-morpholin-4-ylsulfonylamino-N1-[2-(6-oxo-1,6-dihydro-pyridazin-3-ylcarbonylamino-ethyl]-succinamid und

N-[(S)-1-(Amino-imino-methyl)-piperidin-3-ylmethyl]-2[(S)-4-cyclopropyl-1-naphthalen-2-ylsulfonyl-3,6-dioxo-piperazin-2-yl]-acetamid.

Weitere bevorzugte Verbindungen sind folgende:

(S)-N4-[(S)-1-(Amino-imino-methyl)-piperidin-3-ylmethyl]-N1-cyclopropyl-2-morpholin-4-ylacetylamino-N1-(2-pyrazin-2-ylcarbonylamino-ethyl)-succinamid,

3-[[(S)-3[(S)-1-(Amino-imino-methyl)-piperidin-3-ylmethylcarbamoyl]-2-(3-phenyl-propylamino)propionyl]-cyclopropyl-amino]-propionsäure-ethylester,

3-[[(S)-3-[(S)-1-(Amino-imino-methyl)-piperidin-3-ylmethylcarbamoyl]-2-(3-phenyl-propylamino)-propionyl]-cyclopropyl-amino]-propionsäure,

3-[[(S)-3-[(S)-1-(Amino-imino-methyl)-piperidin-3-ylmethylcarbamoyl]-2-morpholin-4-ylsulfonylamino-propionyl]-cyclopropyl-amino]-propionsäure.

Die erfindungsgemässen Verbindungen werden in an sich bekannter Weise dadurch hergestellt, dass man

a) eine Säure der Formel

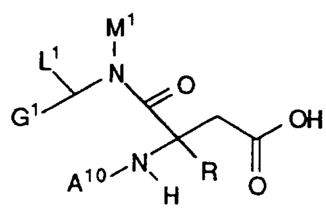

II

mit einem Amin der Formel

$H_2NCH_2Q$ III

oder einem Salz davon gegebenenfalls unter intermediärem Schutz von in den Gruppen $G^1$, $M^1$ und $A^{10}$ -(in II) und Q (in III) enthaltenen funktionellen Gruppen umsetzt oder

b) ein Amin der Formel

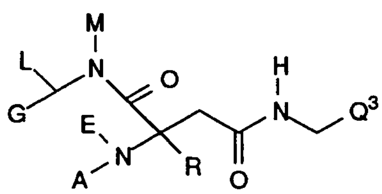

X

worin $Q^3$ eine Gruppe der Formel

($Q^{31}$) 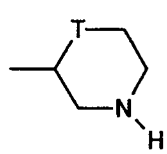 oder ($Q^{32}$) 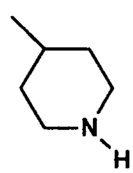

ist,

mit einem die gegebenenfalls hydroxylierte Amidingruppe $-C(NR^7)NHR^6$ einführenden Mittel umsetzt oder

c) ein Amin der Formel

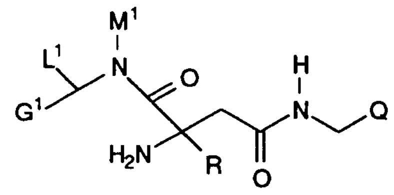

XX

mit einem die Gruppe $A^{10}$ einführenden Mittel umsetzt oder
d) eine Aminosäure der Formel

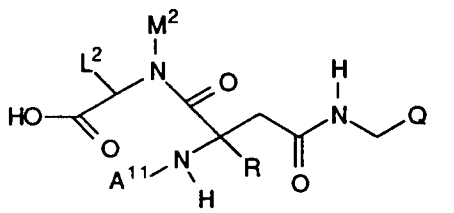

XXX

oder der Formel

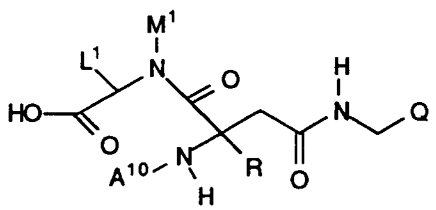

Ia1

zu einer Verbindung der Formel Ib cyclisiert oder
e) dass man zur Herstellung einer Carbonsäure der Formel Ib, worin $M^2$ Carboxy-niederalkyl ist, einen entsprechenden nieder-Alkylester spaltet und
f) gewünschtenfalls eine in einer Verbindung der Formel I enthaltene reaktionsfähige Gruppe funktionell abwandelt, und
g) gewünschtenfalls eine Verbindung der Formel I in ein physiologisch verträgliches Salz überführt oder ein Salz einer Verbindung der Formel I in die freie Säure oder Base überführt.

Zweckmässig wird die Säure II in einem Lösungsmittel, wie Dimethylformamid (DMF) oder Methylenchlorid, in Gegenwart einer Base, wie 4-Aethylmorpholin, Triäthylamin, Aethyldiisopropylamin (Hünig-Base) oder 1,8-Diazabicyclo(5.4.0)undec-7-en (DBU), mit einem Salz einer Verbindung der Formel III, z. B. einem Trifluoracetat, Bisulfit, Nitrat, Hydrochlorid oder Hydrojodid, und mit Benzotriazol-1-yloxy-tris(dimethylamino)phosphonium-hexafluorophosphat (BOP) bei Raumtemperatur umgesetzt. In den Verbindungen II und III enthaltene, intermediär zu schützende funktionelle Gruppen wie COOH, $NH_2$ und OH können in Form von nieder-AlkylOCO-Gruppen, von BenzylOCO- oder Azidgruppen bzw. von Benzyloxygruppen geschützt werden. Die Aufspaltung einer geschützten Carboxygruppe, wie $COOCH_3$ oder $COOC_2H_5$, zu COOH kann mit Natronlauge in Aethanol erfolgen. Die Ueberführung der BenzylOCONH- oder $N_3$-Gruppe in die freie Aminogruppe kann man durch katalytische (Pd/C) Hydrierung in Aethanol durchführen.

Nach der Verfahrensvariante b) kann man zur Herstellung einer Verbindung der Formel I, worin $R^6$ und $R^7$ (in der Gruppe Q) Wasserstoff sind, die entsprechende Verbindung der Formel X in einem Lösungsmittel, wie DMF oder Methanol, in Gegenwart einer Base, wie Triäthylamin, mit Formamidinsulfonsäure oder 3,5-Dimethyl-1-pyrazolyl-formamidinium-nitrat, zweckmässig bei einer Temperatur bis zu 50 °C umsetzen.

Zur Herstellung einer Verbindung I, worin $R^6$ oder $R^7$ (in der Gruppe Q) OH ist, kann eine Verbindung X in einem Lösungsmittel, wie Methylenchlorid, unter Abkühlen mit Bromcyan und dann mit Hydroxylaminhydrochlorid in Gegenwart von einem Amin, wie Triäthylamin, umgesetzt werden.

Zur Durchführung der Variante c) kann man ein Amin XX in einem Lösungsmittel, wie DMF, in Gegenwart von einer Base, wie Ethylmorpholin, mit einer Verbindung der Formel $ClC(O)OR^2$ oder $ClC(O)R^2$, z.B. mit Chlorameisensäure-cyclohexylmethylester, umsetzen. Wird ein Amin XX in einem Lösungsmittel, wie Acetonitril, mit Natriumbicarbonat und einen aktivierten Carbamat, z.B. dem Benzylcarbaminsäure-2,5-dioxopyrrolidin-1-ylester, umgesetzt, so erhält man ein Urethan der Formel Ia, worin $A^{10}$ eine Gruppe -C(O)-$NHR^{22}$ ist. Setzt man ein Amin XX in einem Lösungsmittel, wie Methylenchlorid, mit einer Carbonsäure, z.B. der Pyrimidin-2-thioessigsäure, in Gegenwart einer Base, wie der Hünig-Base, und BOP um, so erhält man eine Verbindung der Formel Ia, in der $A^{10}$ für eine Gruppe -C(O)$R^2$ steht, worin $R^2$ z.B. über nieder-Alkylen gebundenes -(O oder S)-(Aryl, Heteroaryl oder Cycloalkyl) ist.

Die Cyclisation d) kann man in einem Lösungsmittel wie DMF, in Gegenwart einer Base, wie 4-Ethylmorpholin, und BOP durchführen.

Die Spaltung e) eines nieder-Alkylcarbonsäureesters zur entsprechenden Carbonsäure der Formel Ib, worin $M^2$ Carboxy-niederalkyl ist, kann man in einem Lösungsmittel, wie wässrigem Aethanol mit einem Ionentauscher in $Cl^-$-Form bewerkstelligen.

Als funktionelle Abwandlungen in der Variante f) kann man folgende nennen:

1. die Verseifung einer Estergruppe, wie Aethoxycarbonyl, z.B. in THF mittels einer Base, wie wässrigem LiOH;
2. die Abspaltung der Z-Gruppe (Benzyloxycarbonyl) in einer z.B. in der Gruppe G enthaltenen, Z-geschützten Aminogruppe;
3. die Umwandlung der unter 2. erhaltenen freien Aminogruppe G in eine Gruppe NHC(O)-(Aryl oder Heteroaryl) bzw. in eine Gruppe $NHSO_2$-N(Het), z.B. durch Reaktion mit einer Carbonsäure der Formel G-COOH bzw. mit $ClSO_2$-N(Het).

Die weiter oben verwendeten Ausgangsmaterialien lassen sich in an sich bekannter Weise z.B. nach folgenden Reaktionsschemata herstellen:

**Reaktionsschema 1**

(V)

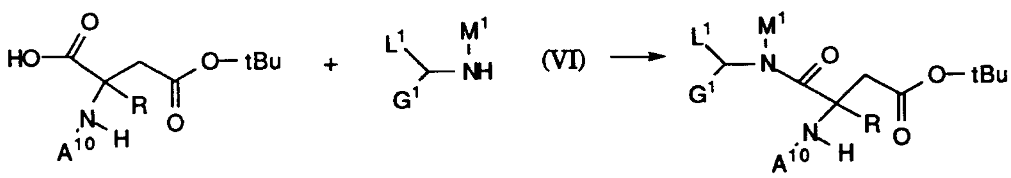

(VI) (IV)

IV + $H_2NCH_2Q$ (III) → II

**Reaktionsschema 2**

(VIII)

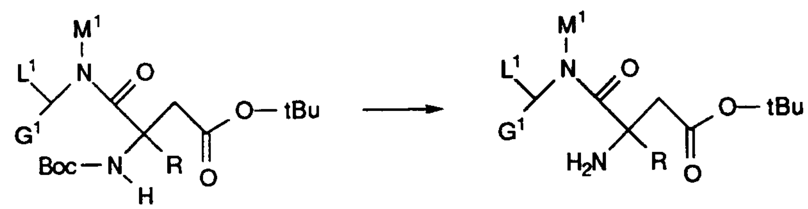

(VII)

VII + $A^{10}Cl$ → IV

Ein Amin XX lässt sich z.B. durch Abspaltung der Z-Gruppe in einer Verbindung der Formel Ia, in der die Gruppe $A^{10}$ eine Benzyloxycarbonylgruppe ist.

Verbindungen XXX sind in der EP-A-468 231 beschrieben. Sie lassen sich wie die Verbindungen der Formel Ia herstellen.

Ausgangsester in der Verfahrensvariante e) kann man wie folgt herstellen:

Reaktionsschema 3

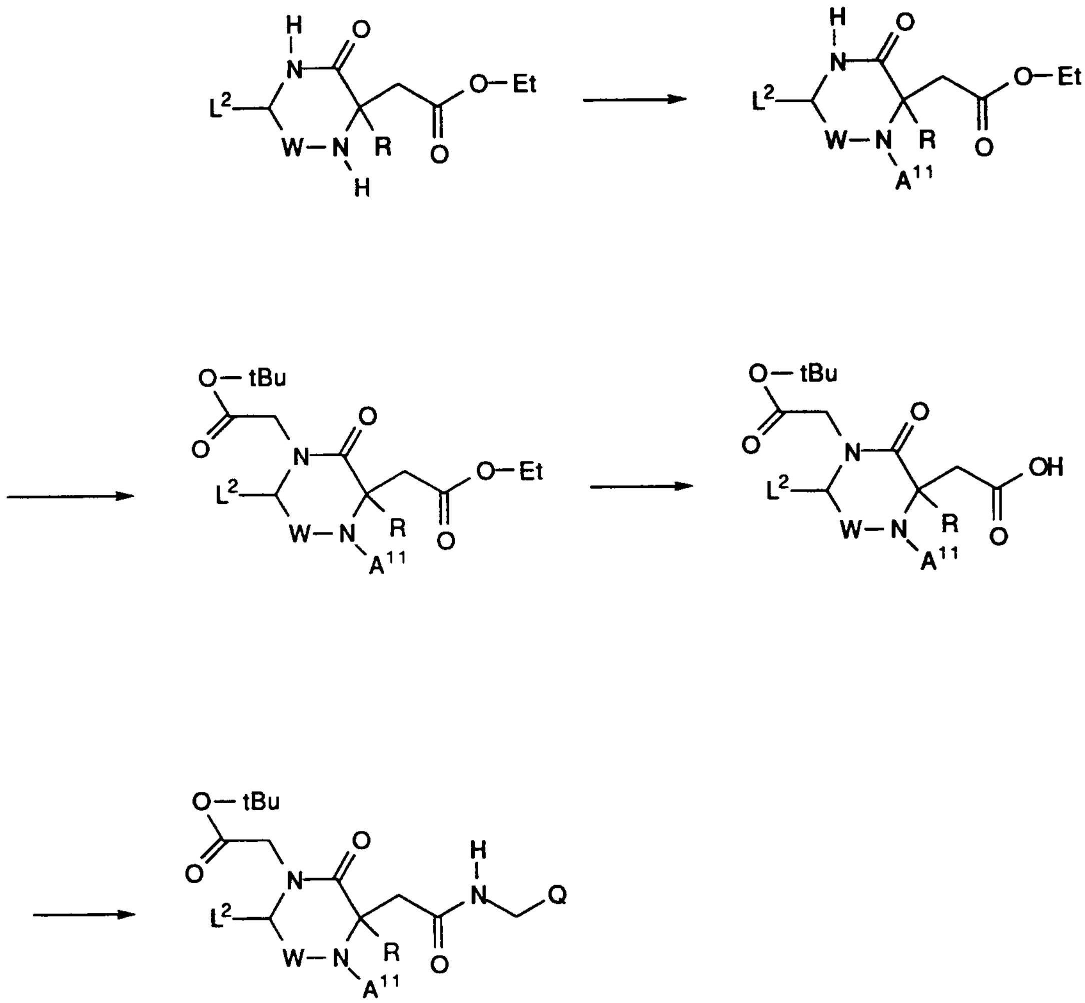

Zudem enthalten manche der nachfolgenden Beispiele detaillierte Angaben betreffend die Herstellung bestimmter Ausgangsmaterialien und Zwischenprodukten.

Die Verbindungen der Formel I, ihre Solvate und ihre Salze hemmen sowohl die durch Thrombin induzierte Plättchenaggregation als auch die durch Thrombin induzierte Gerinnung von Fibrinogen im Blutplasma. Die besagten Verbindungen beeinflussen sowohl die Plättchen induzierte als auch die plasmatische Blutgerinnung. Sie verhindern damit insbesondere die Entstehung von Gerinnungsthromben aber auch von plättchenreichen Thromben und können bei der Bekämpfung bzw. Verhütung von Krankheiten, wie Thrombose, Apoplexie, Herzinfarkt, Entzündung und Arteriosklerose verwendet werden. Ferne haben diese Verbindungen einen Effekt auf Tumorzellen und verhindern die Bildung von Metastasen. Somit können sie auch als Antitumor-Mittel eingesetzt werden.

Eine unterschiedliche Hemmung von Thrombin und anderen Serin-Proteasen durch die obigen Verbindungen ist erstrebenswert, um Verbindungen mit einer möglichst hohen Spezifität zu erhalten und damit mögliche Nebenwirkungen zu vermeiden. Nebst anderen getesteten Serin-Proteasen wurde das Verhältnis der Hemmung von Trypsin zur Hemmung von Thrombin als generelles Mass der Spezifität einer Verbindung genommen (q in der nachstehenden Tabelle), weil Trypsin als unspezifischste Serin-Protease durch verschiedenste Hemmer leicht gehemmt werden kann. Um die Hemmung von Thrombin und Trypsin, trotz Benutzung unterschiedlicher Substrate, direkt vergleichen zu können, wurde als Mass der Hemmung die von Substrat- und Enzymkonzentration unabhängige Hemmkonstante $K_i$ ermittelt.

Zum Nachweis der Hemmung der katalytischen Aktivität der obigen Proteasen können spezifische chromogene Peptidsubstrate benutzt werden. Die Hemmung der amidolytischen Aktivität von Thrombin und Trypsin durch die obigen Verbindungen wurde wie nachstehend beschrieben nachgewiesen.

Die Messungen wurden auf Mikrotiterplatten bei Raumtemperatur durchgeführt. Dafür wurden pro Vertiefung der Platte 150 µl Puffer (50 mM Tris, 100 mM NaCl, 0,1 % Polyaethylenglykol; pH 7,8) mit 50 µl der in DMSO gelösten und im Puffer verdünnten Hemmsubstanz vermischt und 25 µl humanes Thrombin (0,5 nM Endkonz.) zugegeben. Nach 10 Minuten Inkubation wurde die Reaktion durch Zugabe von chromogenem Substrat S-2238 (H-D-Phe-Pip-Arg-Paranitroanilin von Kabivitrum; 10 oder 50 µM Endkonz.) gestartet und die Hydrolyse des Substrates spektrophotometrisch auf einem kinetischen Mikrotiter-Plattenleser während 5 Minuten verfolgt. Nach graphischer Dartstellung der Hemmkurven wurden die $K_i$-Werte nach der in Biochem. J. 55, 1955, 170-171 beschriebenen Methode bestimmt. Die Hemmung von Trypsin erfolgte analog, jedoch unter Verwendung des Substrates S-2251 (H-D-Val-Leu-Lys-Paranitroanilin) in 200 und 750 µM Endkonzentration.

Die Resultate sind aus folgender Tabelle ersichtlich:

| Produkt von Beispiel | $K_i$ (µM) Thrombin | $K_i$ (µM) Trypsin | q |
|---|---|---|---|
| 1 | 1,2 | 70 | 58 |
| 2a | 0,017 | 29 | 1706 |
| 2b | 0,61 | 56 | 92 |
| 2c | 0,077 | 30 | 390 |
| 3 | 0,012 | 19 | 1583 |
| 4a | 0,079 | 83 | 1051 |
| 4b | 0,064 | 28 | 438 |
| 4c | 0,3 | 100 | 333 |
| 5a | 8,9 | 231 | 26 |
| 5b | 0,03 | 150 | 5000 |
| 5c | 0,054 | 29 | 537 |
| 5d | 0,13 | 12 | 92 |
| 6a | 0,45 | 140 | 311 |
| 6b | 0,0035 | 65 | 18571 |
| 6c | 0,33 | 92 | 279 |
| 6d | 0,94 | 85 | 90 |
| 7 | 2 | 50 | 25 |
| 8 | 0,017 | 27 | 1588 |
| 9a | 1,3 | 95 | 73 |
| 9b1 | 1,5 | 80 | 53 |
| 10 | 0,25 | 51 | 204 |
| 11 | 0,043 | 35 | 814 |
| 13a | 2,7 | 70 | 26 |
| 13b | 0,41 | 75 | 183 |
| 13c | | | |
| 13d | 4,7 | 23,5 | 5 |
| 13e | 2,6 | 91 | 35 |
| 14a | 0,019 | 64 | 3368 |
| 14b | 0,036 | 25 | 694 |
| 14c | 0,057 | 50 | 877 |
| 14d | 0,25 | 83 | 332 |
| 14e | 0,081 | 69 | 852 |

| Produkt von Beispiel | $K_i$ (µM) Thrombin | $K_i$ (µM) Trypsin | q |
|---|---|---|---|
| 15 | 0,0018 | 13 | 7222 |
| 16 | 0,11 | 14 | 127 |
| 17 | 1,3 | 96 | 74 |
| 18 | 2,2 | 590 | 268 |
| 19 | 0,0069 | 3,1 | 449 |
| 20 | 0,00086 | 4,5 | 5233 |
| 21a | 0,0023 | 8,9 | 3870 |
| 21b | 0,0072 | 14 | 1944 |
| 21c | 0,0061 | 15 | 2459 |
| 21d | 0,0013 | 25 | 19231 |
| 22 | 0,0015 | 16,5 | 11000 |
| 23 | 0,0046 | 13 | 2826 |
| 24 | 0,0012 | 7,1 | 5917 |
| 25 | 0,0013 | 7,2 | 5538 |

Die Verbindungen der Formel I haben eine geringe Toxizität. So haben die Produkte der in der Tabelle aufgezählten Beispiele bei intravenöser Verabreichung eine LD50 von 125 - 500 mg/kg an der Maus.

Wie eingangs erwähnt, sind Arzneimittel enthaltend eine Verbindung der Formel I, ein Solvat oder Salz davon, ebenfalls Gegenstand der vorliegenden Erfindung, weiterhin auch ein Verfahren zur Herstellung derartiger Arzneimittel, welches dadurch gekennzeichnet ist, dass man eine oder mehrere andere therapeutisch wertvolle Stoffe in galenische Darreichungsform bringt. Die Dragées, Hart- und Weichgelatinekapseln, Lösungen, Emulsionen oder Supensionen, oder rektal, z. B. in Form von Suppositorien, oder als Spray verabreicht werden. Die Verabreichung kann aber auch parenteral, z. B. in Form von Injektionslösungen, erfolgen.

Zur Herstellung von Tabletten, Lacktabletten, Dragées und Hartgelatinekapseln kann der Wirkstoff mit pharmazeutisch inerten, anorganischen oder organischen Excipientien vermischt werden. Als solche Excipientien kann man für Tabletten, Lacktabletten, Dragées und Hartgelatinekapseln z.B. Lactose, Maisstärke oder Derivate davon, Talk, Stearinsäure oder deren Salze, verwenden. Für Weichgelatinekapseln eignen sich als Excipientien z.B. vegetabile Oele, Wachse, Fette, halbfeste und flüssige Polyole; je nach Beschaffenheit des Wirkstoffs sind jedoch bei Weichgelatinekapseln überhaupt keine Excipientien erforderlich. Zur Herstellung der Lösungen und Sirupe eignen sich als Excipientien z.B. Wasser, Polyole, Saccharose, Invertzucker und Glucose, für Injektionslösungen eignen sich z.B. Wasser, Alkohole, Polyole, Glycerin und vegetabile Oele, und für Suppositerien eignen sich natürliche oder gehärtete Oele, Wachse, Fette, halbflüssige oder flüssige Polyole. Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten.

Für die Bekämpfung bzw. Verhütung der weiter oben genannten Krankheiten kann die Dosierung des Wirkstoffes innerhalb weiter Grenzen variieren und ist natürlich in jedem Einzelfall den individuellen Gegebenheiten anzupassen. Im allgemeinen dürfte bei oraler oder parenteraler, z.B. intravenöser oder subkutaner Verabreichung eine Dosis von etwa 0,1 bis 20 mg/kg, vorzugsweise von etwa 0,5 bis 4 mg/kg, pro Tag für den Erwachsenen angemessen sein, wobei aber die soeben angegebene obere Grenze auch über- oder unterschritten werden kann, wenn sich dies als angezeigt erweisen sollte.

Beispiel 1

1.A) 3.6 g (S)-3-Benzyloxycarbonylamino-N-cyclopropyl-N-ethoxycarbonyl-methyl-succinamsäure tert-butylester werden in 36 ml Methylenchlorid gelöst, bei 0° mit 40 ml 4M Salzsäure in Essigester versetzt und 5 Stunden gerührt. Die Lösung wird eingedampft und der farblose Rückstand in 36 ml DMF gelöst, mit 5.1 ml 4-Ethylmorpholin, 3.55 g Benzotriazol-1-yloxy-tris(dimethylamino)phosphonium-hexafluorophosphat (BOP) und 2.02 g (S)-1-Amidino-3-(aminomethyl)piperidin-dihydrochlorid (EP-A-468231: Beispiel 60Ac) versetzt und über Nacht bei Raumtemperatur gerührt. Das Reaktionsgemisch wird eingedampft und der Rückstand mit einem Wasser-Acetonitril Gradienten an RP-18 chromatographiert. Die Produktfraktionen werden eingedampft und mit Wasser-Ethanol über einen Ionentauscher ($Cl^-$ -Form) filtriert. Nach Eindampfen isoliert man farbloses 2.9 g [[(S)-3-[(S)-1-(Amino-imino-methyl)-piperidin-3-ylmethylcarbamoyl]-2-benzyloxycarbonylamino-propionyl]cyclopropyl-amino]-essigsäureethylester-hydrochlorid, MS (Ion-Spray): 531.4 $(M+H)^+$.

1.B) Herstellung der Ausgangsmaterialien:

Zu einer Lösung von 8.0 g N-Z-L-Asparaginsäure-β-tert-butylester in 80 ml Methylenchlorid gibt man nacheinander 5.0 g N-(Dimethylaminopropyl)-N'-ethylcarbodiimid-hydrochlorid, 0.3 g 4-Dimethylaminopyridin und 3.7 g N-Cyclopropylglycinethylester und rührt 17 Stunden bei Raumtemperatur. Dann wird das Reaktionsgemisch auf eiskalte 5%ige Kaliumhydrogensulfat-10%ige Kaliumsulfat Lösung gegossen und mit Essigester extrahiert. Die organischen Phasen werden mit Wasser gewaschen, getrocknet, eingedampft und der Rückstand mit Hexan-Essigester (3:1) an Kieselgel chromatographiert. Man erhält 8.4 g farbloses, öliges (S)-3-Benzyloxycarbonylamino-N-cyclopropyl-N-ethoxycarbonylmethyl-succinamsäure-tert-butylester, MS (FAB): 449.2 $(M^+)$.

Beispiel 2

2.A) Analog Beispiel 1 stellt man folgende Verbindungen her:

a) 3-[[(S)-3-[(S)-1-(Amino-imino-methyl)-piperidin-3-ylmethylcarbamoyl]-2-benzyloxycarbonylamino-propionyl]-cyclopropyl-amino]-propionsäureethylester-hydrochlorid, MS (Ion-Spray): 545.4 $(M+H)^+$,
b) [[(S)-3-[(S)-1-(Amino-imino-methyl)-piperidin-3-ylmethylcarbamoyl]-2-benzyloxycarbonylamino-propionyl]-butyl-amino]-essigsäureethylester-hydrochlorid, MS (Ion-Spray): 547.5 $(M+H)^+$,
c) 3-[[(S)-3-[(S)-1-(Amino-imino-methyl)-piperidin-3-ylmethylcarbamoyl]-2-benzyloxycarbonylamino-propionyl]-butyl-amino]-propionsäureethylester-hydrochlorid, MS (Ion-Spray): 561.4 $(M+H)^+$.

2.B) Herstellung der Ausgangsmaterialien:

Analog Beispiel 1.B) jedoch unter Verwendung von N-Cyclopropyl-β-alaninethylester, N-Butylglycinethylester bzw. N-Butyl-β-alaninethylester anstelle von N-Cyclopropylglycinethylester stellt man folgende Zwischenprodukte her:
a) (S)-3-Benzylcarbonylamino-N-cyclopropyl-N-(2-ethoxycarbonyl-ethyl)-succinamsäure-tert-butylester, MS (FAB): 463.3 $(M+H)^+$,
b) (S)-3-Benzylcarbonylamino-N-butyl-N-ethoxycarbonylmethyl-succinamsäure-tert-butylester, MS (Ion-Spray): 465.4 $(M+H)^+$,
c) (S)-3-Benzylcarbonylamino-N-butyl-N-(2-ethoxycarbonyl-ethyl)-succinamsäure tert-butylester, MS (FAB): 479.3 $(M+H)^+$.

Beispiel 3

1.5 g [[(S)-3-[(S)-1-(Amino-imino-methyl)-piperidin-3-ylmethylcarbamoyl]-2-benzyloxycarbonylaminopropionyl]-cyclopropyl-amino]-essigsäureethylester-hydrochlorid (Beispiel 1) werden in 15 ml THF gelöst, mit 7.9 ml 1N Lithiumhydroxid-Lösung versetzt und 90 Minuten bei Raumtemperatur gerührt. Dann wird die Lösung mit 7.9 ml 1N Salzsäure versetzt und eingedampft. Der Rückstand wird mit einem Wasser-Acetonitril Gradienten an RP-18 chromatographiert. Man erhält 0.63 g farblose 1.5 g [[(S)-3-[(S)-1-(Amino-imino-methyl)-piperidin-3-ylmethylcarbamoyl]-2-benzyloxycarbonyl-amino-propionyl]-cyclopropyl-amino]-essigsäure,MS (Ion-Spray): 503.5 $(M+H)^+$.

Beispiel 4

Analog Beispiel 3 jedoch ausgehend von den Esterprodukten von Beispiel 2 erhält man die folgenden Säuren:

a) 3-[[(S)-3-[(S)-1-(Amino-imino-methyl)-piperidin-3-ylmethylcarbamoyl]-2-benzyloxycarbonylamino-propionyl]-cyclopropyl-amino]-propionsäure, MS (Ion-Spray): 517.4 $(M+H)^+$,

b) [[(S)-3-[(S)-1-(Amino-imino-methyl)-piperidin-3-ylmethylcarbamoyl]-2-benzyloxycarbonylamino-propionyl]-butyl-amino]-essigsäure, MS (Ion-Spray): 519.4 $(M+H)^+$,

c) 3-[(S)-3-[(S)-1-(Amino-imino-methyl)-piperidin-3-ylmethylcarbamoyl]-2-benzyloxycarbonylamino-propionyl]-butyl-amino]-propionsäure,MS (Ion-Spray): 533.5 $(M+H)^+$.

Beispiel 5

Analog Beispiel 1 jedoch unter Verwendung von rac-2-Aminomethyl-4-morpholinecarboxamidin-dihydrochlorid anstelle des (S)-1-Amidino-3-(aminomethyl)-piperidin-dihydrochlorids werden folgende Verbindungen hergestellt:

a) aus (S)-3-Benzyloxycarbonylamino-N-cyclopropyl-N-ethoxycarbonyl-methyl-succinamsäure tert-butylester das [[(S)-3-[4-(Amino-imino-methyl)-morpholin-2-ylmethylcarbamoyl]-2-benzyloxycarbonylamino-propionyl]-cyclopropyl-amino]-essigsäureethylester-hydrochlorid (1:1), MS (Ion-Spray): 533.4 $(M+H)^+$,

b) aus (S)-3-Benzyloxycarbonylamino-N-butyl-N-ethoxycarbonylmethyl-succinamsäure tert-butylester das [[(S)-3-[4-(Amino-imino-methyl)-morpholin-2-ylmethylcarbamoyl]-2-benzyloxycarbonylamino-propionyl]-butyl-amino]-essigsäureethylester-hydrochlorid (1:1), MS (Ion-Spray): 549.4 $(M+H)^+$,

c) aus (S)-3-Benzyloxycarbonylamino-N-cyclopropyl-N-(2-ethoxycarbonyl-ethyl)-succinamsäure tert-butylester das 3-[[(S)-3-[4-(Amino-imino-methyl)-morpholin-2-ylmethylcarbamoyl]-2-benzyloxycarbonylamino-propionyl]-cyclopropyl-amino]-propionsäureethylester-hydrochlorid (1:1), MS (Ion-Spray): 547.5 $(M+H)^+$,

d) aus (S)-3-Benzyloxycarbonylamino-N-butyl-N-(2-ethoxycarbonyl-ethyl)-succinamsäure tert-butylester das 3-[[(S)-3-[4-(Amino-imino-methyl)-morpholin-2-ylmethylcarbamoyl]-2-benzyloxycarbonylamino-propionyl]-butyl-amino]-propionsäureethylester-hydrochlorid (1:1), MS (Ion-Spray): 563.5 $(M+H)^+$.

Beispiel 6

Analog Beispiel 3 jedoch ausgehend von den Estern von Beispiel 5 erhält man die folgenden Säuren:

a) [[(S)-3-[4-(Amino-imino-methyl)-morpholin-2-ylmethylcarbamoyl]-2-benzyloxycarbonylamino-propionyl]-cyclopropyl-amino]essigsäure (1:1), MS (Ion-Spray): 505.0 $(M+H)^+$,

b) [[(S)-3-[4-(Amino-imino-methyl)-morpholin-2-ylmethylcarbamoyl]-2-benzyloxycarbonylamino-propionyl]-butyl-amino]-essigsäure (1:1), MS (Ion-Spray): 521.1 $(M+H)^+$,

c) 3-[[(S)-3-[4-(Amino-imino-methyl)-morpholin-2-ylmethylcarbamoyl]-2-benzyloxycarbonylamino-propionyl]-cyclopropyl-amino]-propionsäure(1:1), MS (Ion-Spray): 519.4 $(M+H)^+$,

d) 3-[[(S)-3-[4-(Amino-imino-methyl)-morpholin-2-ylmethylcarbamoyl]-2-benzyloxycarbonylamino-propionyl]-butyl-amino]-propionsäure (1:1), MS (Ion-Spray): 535.4 $(M+H)^+$.

Beispiel 7

7.A) 4.4. g des Produktes von Beispiel 1.A) werden in 44 ml Ethanol gelöst, mit 7.7 ml 1N Salzsäure und 0.44g Pd/C versetzt und 1 Stunde bei Raumtemperatur hydriert. Nach Filtration und eindampfen der Lösung erhält man 3.4 g farbloses [[(S)-2-Amino-3-[(S)-1-(amino-imino-methyl)-piperidin-3-ylmethylcarbamoyl]-propionyl]-cyclopropyl-amino]-essigsäureethylester-dihydrochlorid, MS (Ion-Spray): 397.4 $(M+H)^+$.

7.B) Analog Beispiel 7.A) jedoch ausgehend von den Produkten von Beispiel 2.A) erhält man folgende Amine:

7.B)a) 3-[[(S)-2-Amino-3-[(S)-1-(amino-imino-methyl)-piperidin-3-ylmethyl-carbamoyl]-propionyl]-cyclopropyl-amino]-propionsäureethylester-dihydrochlorid,

7.B)b) 3-[[(S)-2-Amino-3-[(S)-1-(amino-imino-methyl)-piperidin-3-ylmethylcarbamoyl]-propionyl]-butyl-amino]-essigsäureethylester-dihydrochlorid,

7.B)c) 3-[[(S)-2-Amino-3-[(S)-1-(amino-imino-methyl)-piperidin-3-ylmethylcarbamoyl]-propionyl]-butyl-amino]-propionsäureethylester-dihydrocblorid, MS (Ion-Spray): 427.4 $(M+H)^+$.

7.C) Analog Beispiel 7.A) jedoch ausgehend von dem Produkt von Beispiel 3 bzw. 4c) erhält man folgende Amine:

7.C)a) [[(S)-2-Amino-3-[(S)-1-(amino-imino-methyl)-piperidin-3-ylmethylcarbamoyl]-propionyl]-cyclopropyl-amino]-essigsäure-hydrochlorid, MS (Ion-Spray): 369.4 $(M+H)^+$,
7.C)b) 3-[[(S)-2-Amino-3-[(S)-1-(amino-imino-methyl)-piperidin-3-ylmethylcarbamoyl]-propionyl]-butyl-amino]-propionsäure-hydrochlorid, MS (Ion-Spray): 399.4 $(M+H)^+$.
7.D) Zu einer Lösung von 1.7 g des Aminproduktes von Beispiel 7.A) in 18 ml DMF werden 2.4 ml 4-Ethylmorpholin und 0.73 g Chlorameisensäurecyclohexylmethylester gegeben und über Nacht bei Raumtemperatur gerührt. Das Reaktionsgemisch wird eingedampft, mit 1N Salzsäure versetzt und wiederum eingedampft und der Rückstand mit einem Acetonitril-Wasser Gradienten an RP-18 chromatographiert. Nach Eindampfen der Produktfraktionen erhält man 1.6 g [[(S)-3-[(S)-1-(Amino-imino-methyl)-piperidin-3-ylmethylcarbamoyl]-2-cyclohexylmethoxy-carbonylamino-propionyl]-cyclopropyl-amino]-essigsäureethylester-hydrochlorid, MS (Ion-Spray): 537.6 $(M+H)^+$.

Beispiel 8

Analog Beispiel 3 jedoch ausgehend von dem Ester von Beispiel 7.D) erhält man die folgende Säure:
[[(S)-3-[(S)-1-(Amino-imino-methyl)-piperidin-3-ylmethylcarbamoyl]-2-cyclohexylmethoxycarbonylamino-propionyl]-cyclopropyl-amino]-essigsäure, MS (Ion-Spray): 509.5 $(M+H)^+$.

Beispiel 9

9.A) Herstellung der Produkte:

9.A)a) 1.7 g des Aminproduktes von Beispiel 7.A) werden in 17 ml Acetonitril und 17 ml Wasser gelöst. Man gibt nacheinander 0.6 g Natriumbicarbonat und 1.35 g Benzylcarbaminsäure-2,5-dioxo-pyrrolidin-1-ylester zu und rührt 17 Stunden bei Raumtemperatur. Das Reaktionsgemisch wird eingedampft und der Rückstand auf einer RP-18 Säule mit einem Acetonitril-Wasser Gradienten gereinigt. Man isoliert 0.6 g farbloses [[(S)-3-[(S)-1-(Amino-imino-methyl)-piperidin-3-ylmethylcarbamoyl]-2-(3-benzyl-ureido)-propionyl]-cyclopropyl-amino]-essigsäureethylester-hydrochlorid, MS (Ion-Spray): 530.5 $(M+H)^+$.
9.A)b) In analoger Weise jedoch unter Verwendung der entsprechenden aktivierten Carbamate werden die folgenden Verbindungen hergestellt:
9.A)b)1) [[(S)-3-[(S)-1-(Amino-imino-methyl)-piperidin-3-ylmethylcarbamoyl]-2-(3-furan-2-ylmethyl-ureido)-propionyl]-cyclopropyl-amino]-essigsäureethylester-hydrochlorid, MS (Ion-Spray): 520.5 $(M+H)^+$,
9.A)b)2) [[(S)-3-[(S)-1-(Amino-imino-methyl)-piperidin-3-ylmethylcarbamoyl]-2-(3-butyl-ureido)-propionyl]-cyclopropyl-amino]-essigsäureethylester-hydrochlorid, MS (Ion-Spray): 496.6 $(M+H)^+$.

9.B) Herstellung des Ausgangsmaterials:

9.B)a) Zu einer Lösung von 6.4 g Benzylamin in 150 ml Acetonitril werden bei 5°C innert 5 Minuten 18.4 g N,N'-Disuccinimidylcarbonat in kleinen Portionen zugegeben. Das Reaktionsgemisch wird bei Raumtemperatur gerührt und dann eingedampft. Der Rückstand wird in Essigester aufgenommen und mit Wasser gewaschen. Die organische Phase wird getrocknet, eingeengt und der Rückstand aus Methylenchlorid-Hexan umkristallisiert. Man isoliert 11.3 g farbloses Benzylcarbaminsäure-2,5-dioxo-pyrrolidin-1-ylester, MS (FAB): 133 (M- HO-Succ).
9.B)b) In analoger Weise jedoch unter Verwendung der entsprechenden Amine anstelle von Benzylamin werden die folgenden aktivierten Carbamate hergestellt:
9.B)b)1) Furan-2-ylmethyl-carbaminsäure-2,5-dioxo-tetrahydropyrrol-1-ylester, MS (FAB): 123 (M- HO-Succ),
9.B)b)2) Pyridin-3-ylmethyl-carbaminsäure-2,5-dioxo-pyrrolidin-1-ylester, MS (FAB): 151 (M- Succ),
9.B)b)3) Butyl-carbaminsäure-2,5-dioxo-pyrrolidin-1-ylester, MS (FAB): 214 (M).

Beispiel 10

Analog Beispiel 9, jedoch ausgehend von dem Amin aus Absatz 7.B)c) und unter Verwendung des aktivierten Esters von Absatz 9.B)b)1 wird folgender Ester hergestellt:
3-[[(S)-3-[(S)-1-(Amino-imino-methyl)-piperidin-3-ylmethylcarbamoyl]-2-(3-furan-2-ylmethyl-ureido)-propionyl]-butyl-amino]-propionsäureethylester-hydrochlorid (1:1), MS (Ion-Spray): 550.4 $(M+H)^+$.

Beispiel 11

2.5 g des Produktes von Beispiel 7.C)a) werden in 25 ml Acetonitril und 25 ml Wasser gelöst. Man gibt nacheinander 1.0 g Natriumbicarbonat und 2.3 g Benzylcarbaminsäure-2,5-dioxo-pyrrolidin-1-ylester zu und rührt 5 Stunden bei Raumtemperatur. Das Reaktionsgemisch wird eingedampft und der Rückstand auf einer RP-18 Säule mit einem Acetonitril-Wasser Gradienten gereinigt. Man isoliert 0.8 g farbloses [[(S)-3-[(S)-1-(Amino-imino-methyl)-piperidin-3-ylmethylcarbamoyl]-2-(3-benzyl-ureido)-propionyl]-cyclopropyl-amino]-essigsäure, MS (Ion-Spray): 502.4 $(M+H)^+$.

Beispiel 12

Analog Beispiel 3 jedoch ausgehend von den Estern aus Beispiel 9.A)b)1), 9.A)b)2) und 10 werden folgende Säuren hergestellt:

a) [[(S)-[(S)-1-(Amino-imino-methyl)-piperidin-3-ylmethylcarbamoyl]-2-(3-furan-2-ylmethyl-ureido)-propionyl]-cyclopropyl-amino]-essigsäure, MS (Ion-Spray): 492.5 $(M+H)^+$,

b) [[(S)-3-[(S)-1-(Amino-imino-methyl)-piperidin-3-ylmethylcarbamoyl]-2-(3-n-butyl-ureido)-propionyl]-cyclopropyl-amino]-essigsäure, MS (Ion-Spray): 468.4 $(M+H)^+$,

c) [[(S)-[(S)-1-(Amino-imino-methyl)-piperidin-3-ylmethylcarbamoyl]-2-(3-furan-2-ylmethyl)-ureido)-propionyl]-n-butyl-amino]-propionsäure, MS (Ion-Spray): 522.4 $(M+H)^+$.

Beispiel 13

A) Analog Beispiel 1 stellt man folgende Verbindungen her:

a) [[(S)-3-[(S)-1-(Amino-imino-methyl)-piperidin-3-ylmethylcarbamoyl]-2-pentanoylamino-propionyl]-cyclopropyl-amino]-essigsäureethylester-hydrochlorid, MS (Ion-Spray): 481.5 $(M+H)^+$,

b) [[(S)-3-[(S)-1-(Amino-imino-methyl)-piperidin-3-ylmethylcarbamoyl]-2-hexanoylamino-propionyl]-cyclopropyl-amino]-essigsäureethylester-hydrochlorid, MS (Ion-Spray): 495.6 $(M+H)^+$,

c) [[(S)-3-[(S)-1-(Amino-imino-methyl)-piperidin-3-ylmethylcarbamoyl]-2-(4-chloro-pyridin-2-ylcarbonylamino)-propionyl]-cyclopropylamino]-essigsäureethylester-hydrochlorid, MS (Ion-Spray): 536.5 $(M+H)^+$,

d) [[(S)-3-[(S)-1-(Amino-imino-methyl)-piperidin-3-ylmethylcarbamoyl]-2-benzoylamino-propionyl]-cyclopropyl-amino]-essigsäureethylester-hydrochlorid, MS (Ion-Spray): 501.6 $(M+H)^+$,

e) [[(S)-3-[(S)-1-(Amino-imino-methyl)-piperidin-3-ylmethylcarbamoyl]-2-benzyloxycarbonylamino-propionyl]-cyclopropyl-amino]-essigsäureethylester-hydrochlorid, MS (Ion-Spray): 515.5 $(M+H)^+$.

B) Herstellung der Ausgangsmaterialien:

a) Eine Lösung von 16.6 g t-Butyl (S)-2-(2-tert-butoxyformamido)-N-cyclopropyl-N-[(ethoxycarbonyl)-methyl]succinamat (analog hergestellt wie das Produkt von Beispiel 1B) in 170 ml Dioxan wird mit 19.0 g p-Toluolsulfonsäure-monohydrat versetzt und während 30 Stunden bei Raumtemperatur gerührt. Dann wird die Lösung unter Kühlen mit 17.8 ml Pyridin und anschliessend mit 9.6 ml Valeroylchlorid versetzt und über Nacht bei Raumtemperatur gerührt. Zur Aufarbeitung wird das Reaktionsgemisch auf eiskalte 5%ige Kaliumhydrogensulfat-10%ige Kaliumsulfat Lösung gegossen und mit Essigester extrahiert. Die organischen Phasen werden mit verdünnter Kochsalzlösung gewaschen, getrocknet und eingedampft. Nach Chromatografie des Rückstands an Kieselgel mit Hexan-Essigester (2:1) isoliert man 4.8 g farblosen (S)-N-Cyclopropyl-N-ethoxycarbonylmethyl-3-pentanoylamino-succinamsäure-tert-butylester, MS (Ion-Spray): 399.4 $(M+H)^+$.

b) Analog werden unter Verwendung der entsprechenden Säurechloride anstelle von Valeroylchlorid die folgenden t-Butylester hergestellt:

b)1) (S)-N-Cyclopropyl-N-ethoxycarbonylmethyl-3-hexanoylamino-succinamsäure-tert-butylester, MS (FAB): 413 $(M+H)^+$,

b)2) [[(S)-3-tert-Butoxycarbonyl-2-(4-chloro-pyridin-2-ylcarbonylamino)-propionyl]-cyclopropyl-amino]-essigsäureethylester, MS (FAB): 213 $(M+H)^+$,

b)3) (S)-3-Benzoylamino-N-cyclopropyl-N-ethoxycarbonylmethyl-succinamsäure-tert-butylester, MS (FAB): 419 $(M+H)^+$,

b)4) (S)-2-Benzylcarbonylamino-N-cyclopropyl-N-ethoxycarbonylmethyl-succinamsäure-tert-butylester, MS (Ion-Spray): 433.1 $(M+H)^+$.

Beispiel 14

Analog Beispiel 3 jedoch ausgehend von den Estern von Beispiel 13A) erhält man die folgenden Säuren:

a) [[(S)-3-[(S)-1-(Amino-imino-methyl)-piperidin-3-ylmethylcarbamoyl]-2-pentanoylamino-propionyl]-cyclopropyl-amino]-essigsäure, MS (Ion-Spray): 453.4 $(M+H)^+$,

b) [[(S)-3-[(S)-1-(Amino-imino-methyl)-piperidin-3-ylmethylcarbamoyl]-2-hexanoylamino-propionyl]-cyclopropyl-amino]-essigsäure, MS (Ion-Spray): 467.4 $(M+H)^+$,

c) [[(S)-3-[(S)-1-(Amino-imino-methyl)-piperidin-3-ylmethylcarbamoyl]-2-(4-chloro-pyridin-2-ylcarbonylamino)-propionyl]-cyclopropyl-amino]-essigsäure, MS (Ion-Spray): 508.5 $(M+H)^+$,

d) [[(S)-3-[(S)-1-(Amino-imino-methyl)-piperidin-3-ylmethylcarbamoyl]-2-benzoylamino-propionyl]-cyclopropyl-amino]-essigsäure, MS (Ion-Spray): 473.6 $(M+H)^+$,

e) [[(S)-3-[(S)-1-(Amino-imino-methyl)-piperidin-3-ylmethylcarbamoyl]-2-benzylcarbonylamino-propionyl]-cyclopropyl-amino]-essigsäure,MS (Ion-Spray): 487.4 $(M+H)^+$.

Beispiel 15

Eine Lösung von N-[N4-[[(S)-1-Amidino-3-piperidinyl]methyl]-N2-(naphthalen-2-ylsulfonyl)-L-asparaginyl]-N-cyclopropylglycin

-(herstellbar aus N-Boc-L-Asparaginsäure-$\beta$-t-butylester und N-Cyclopropylglycinäthylester, via t-Butyl-(S)-3(1-t-butoxyformamido)-N-cyclopropyl-N-[(äthyloxycarbonyl)methyl]succinamat und via t-Butyl-(S)-N-cyclopropyl-N-[(äthoxycarbonyl)methyl]-3-(naphthalen-2-ylsulfonamido)-succinamat)-

in 30 ml DMF werden mit 1.7 ml 4-Ethylmorpholin und 1.2 g BOP versetzt und über Nacht bei Raumtemperatur gerührt. Dann wird das Reaktionsgemisch eingedampft, mit 1N Salzsäure versetzt und wiederum eingedampft. Der Rückstand wird mit einem Acetonitril-Wasser Gradienten an RP-18 chromatographiert. Die Produkt enthaltenden Fraktionen werden eingeengt und man isoliert 0.7 g N-[(S)-1-(Amino-imino-methyl)-piperidin-3-ylmethyl]-2[(S)-4-cyclopropyl-1-naphthalen-2-ylsulfonyl-3,6-dioxo-piperazin-2-yl]-acetamid-hydrochlorid, MS (Ion-Spray): 541.5 $(M+H)^+$.

Beispiel 16

In zu Beispiel 15 analoger Weise erhält man aus:

(S)-N4-[(S)-1-(Amino-imino-methyl)-piperidin-3-ylmethyl]-N1-carboxy-methyl-N1-cyclopentyl-2-(naphthalin-2-sulfonylamino)-succinamid

-(herstellbar aus N-Boc-L-Asparaginsäure-$\beta$-t-butylester und N-Cyclopentylglycinäthylester, via t-Butyl-(S)-3(1-t-butoxyformamido)-N-cyclopentyl-N-[(äthyloxycarbonyl)methyl]succinamat und via t-Butyl-(S)-N-cyclopentyl-N-[(äthoxycarbonyl)methyl]-3-(naphthalen-2-ylsulfonamido)succinamat)-

das N-[(S)-1-(Amino-imino-methyl)-piperidin-3-ylmethyl]-2[(S)-4-cyclopentyl-1-naphthalen-2-ylsulfonyl-3,6-dioxo-piperazin-2-yl]-acetamid-hydrochlorid, MS (Ion-Spray): 569.4 $(M+H)^+$.

Beispiel 17

A) Herstellung des Produkts:

0.8 g (RS)-(4-tert-Butoxycarbonylmethyl-1-butylsulfonyl-3-oxo-piperazin-2-yl)-essigsäure werden in 15 ml DMF gelöst, nacheinander mit 1.3 ml 4-Ethylmorpholin, 0.92 g BOP und 0.72 g (S)-1-Amidino-3-(aminomethyl)-piperidin-dihydrochlorid versetzt. Das Reaktionsgemisch wird über Nacht bei Raumtemperatur gerührt, dann eingedampft und der Rückstand mit einem Wasser-Acetonitril Gradienten an RP-18 chromatographiert. Die Produktfraktionen werden eingedampft und mit Wasser-Ethanol über einen Ionentauscher ($Cl^-$-Form) filtriert. Nach Eindampfen isoliert man farbloses [3-[(S)-1-(Amino-imino-methyl)-piperidin-3-ylmethylcarbamoylmethyl]-4-butylsulfonyl-2-oxo-piperazin-1-yl]essigsäure (1:1), MS (Ion-Spray): 475.6 $(M+H)^+$.

B) Herstellung des Ausgangsmaterials:

a) 15.0 g Ethyl-2-piperazin-3-on-acetat werden in 150 ml Pyridin gelöst und bei Raumtemperatur mit 12.5 ml 1-Butansulfochlorid versetzt. Man rührt über Nacht bei Raumtemperatur und dampft dann das Reaktionsgemisch ein. Nach Chromatografie des Rückstandes an Kieselgel mit Essigester-Hexan erhält

man 23.1 g farbloses (RS)-(1-Butylsulfonyl-3-oxo-piperazin-2-yl)-essigsäureethylester, MS (FAB): 261 (M-OEt).

b) Zu einer Lösung von 2.0 g des unter a) erhaltenen Materials in 20 ml DMF gibt man bei -15°C nacheinander 520 mg Natriumhydrid-Dispersion (60%ig) und eine Lösung von 1.9 ml Bromessigsäure-tert-butylester in 30 ml DMF. Man rührt das Reaktionsgemisch bei Raumtemperatur, versetzt es dann mit Wasser und extrahiert mit Essigester. Die organischen Phasen werden mit Wasser gewaschen, getrocknet und eingedampft. Der Rückstand wird mit Essigester-Hexan an Kieselgel chromatographiert und man isoliert 1.8 g farbloses (RS)-3-Ethoxycarbonyl-2-oxo-4-butylsulfonyl-piperazin-1-ylessigsäure tert-butylester, MS (FAB): 375 (M-OEt).

c) 1.7 g des unter b) erhaltenen Materials werden in 17 ml THF gelöst, mit 12 ml 1N Lithiumhydroxid-Lösung versetzt und 2 Stunden bei Raumtemperatur gerührt. Dann werden 10 ml Essigsäure zugegeben und das Reaktionsgemisch eingedampft. Der Rückstand wird in Essigester aufgenommen, mit eiskalter 5%ige Kaliumhydrogensulfat-10%ige Kaliumsulfat Lösung und mit Wasser gewaschen. Die organischen Phasen werden getrocknet und eingedampft. Man erhält 0.8 g (RS)-(4-tert-Butoxy-carbonylmethyl-1-butylsulfonyl-3-oxo-piperazin-2-yl)-essigsäure, MS (Ion-Spray): 391.3 $(M-H)^-$.

Beispiel 18

Eine Lösung von 1.3 g [[(S)-3-[(S)-1-(Amino-imino-methyl)-piperidin-3-ylmethylcarbamoyl]-2-benzyloxycarbonylaminopropionyl]-butyl-amino]-essigsäureethylester-hydrochlorid (Beispiel 2.A)b) werden in 13 ml Ethanol und 13 ml 1N Salzsäure gelöst, mit 0.13 g Pd/C versetzt und 90 Minuten bei Raumtemperatur hydriert. Nach Filtration des Reaktionsgemisches, Eindampfen des Filtrates und Chromatographie des Rückstandes mit einem Acetonitril-Wasser Gradienten an RP-18 isoliert man 0.4 g N-[(S)-1-(Amino-imino-methyl)-piperidin-3-ylmethyl]-2-[(S)-2,6-dioxo-4-butyl-piperazin-2-yl]-acetamid-hydrochlorid, MS (Ion-Spray): 367.4 $(M+H^+$.

Beispiel 19

4.1 g Cyclopropyl-2-(pyrazin-2-ylcarbonylamino)ethylamin-hydrochlorid (1:2), 4.98 g Z-Asp(OtBu)-OH und 7.37 g BOP werden in 100 ml Methylenchlorid gelöst und unter Rühren mit 10.06 ml Hünig's Base versetzt. Nach 2 Stunden Rühren bei Raumtemperatur wird das Gemisch in Aether aufgenommen und mit Wasser gewaschen. Nach Eindampfen der Essigesterphase und Chromatographie des Rückstandes an Kieselgel erhält man 7.06 g (S)-2-Benzyloxycarbonylamino-N1-cyclopropyl-N1-(2-pyrazin-2-ylcarbonylamino-ethyl)-succinamsäure-tert-butylester, MS (ISP): 512 (M+H).

Von den 7.06 g (S)-2-Benzyloxycarbonylamino-N1-cyclopropyl-N1-(2-pyrazin-2-ylcarbonylamino-ethyl)-succinamsäure-tert-butylester löst man 5.92 g in 100 ml Salzsäure (in Essigester) und rührt das Reaktionsgemisch bei Raumtemperatur. Nach Abdampfen des Essigesters löst man in 70 ml Methylenchlorid und gibt 3.96 ml Hünig's Base und 2.52 g Di-tert-butyl-dicarbonat zu und rührt das Gemisch bei Raumtemperatur. Das Gemisch wird eingeengt, der Rückstand wird in 70 ml Methylenchlorid gelöst und man gibt unter Rühren nacheinander 2.75 g (S)-1-Amidino-3-(amino-methyl)piperidin-dihydrochlorid, 10.27 ml Hünig's Base und 5.31 g BOP zu und rührt bei Raumtemperatur. Den Eindampfrückstand chromatographiert man über Kieselgel mit Essigester-Aceton-Wasser-Eisessig 6:2:1:1. Man erhält 3.17 g reines (S)-N4-[(S)-1-(Amino-imino-methyl)-piperidin-3-ylmethyl]-2-tert-butoxycarbonylamino-N1-cyclopropyl-N1-(2-pyrazin-2-ylcarbonylamino)-succinamid-acetat (1:2), MS (ISP): 560.5 (M+H).

Herstellung des Ausgangsmaterials:

5 g 2-Cyclopropylamino-ethylcarbaminsäurebenzylester-hydrochlorid (1:1) (herstellbar aus Z-Glycin und Cyclopropylamin via Benzyloxycarbonylamino-essigsäurecyclopropylamid) werden bei Raumtemperatur unter Rühren mit 4.36 g Di-tert-butyl-dicarbonat, 3.08 ml Triäthylamin und 100 mg Dimethylaminopyridin versetzt. Nach 20 Stunden Rühren bei Raumtemperatur nimmt man das Gemisch in Aether auf und wäscht die Aetherphase mit 1N Salzsäure und Wasser. Die Aetherphase engt man ein. Das erhaltene Oel wird in 100 ml Methanol gelöst und mit 200 mg Pd/C (10%) versetzt und hydriert. Nach Abfiltrieren des Katalysators engt man die Methanollösung ein. Eine Lösung des Rückstands in 100 ml Methylenchlorid versetzt man mit 2.34 g Pyrazincarbonsäure, 9.5 ml Hünig's Base und 8.44 g BOP. Nach Rühren bei Raumtemperatur nimmt man das Gemisch in Aether auf und wäscht die Aetherphasen mit Wasser. Den Rückstand chromatographiert man über Kieselgel mit Essigester-Hexan 1:1. Das erhaltene Oel wird in 40 ml Aether gelöst und mit 30 ml 5N Salzsäure (in Dioxan) versetzt. Nach Rühren gibt man Aether zu und filtriert

die ausgefallenen Kristalle ab. Man erhält 4.1 g weisses kristallines Cyclopropyl-2-(pyrazin-2-ylcarbonylamino)ethylamin-hydrochlorid (1:2), MS (EI): 207 (M + H).

Beispiel 20

300 mg (S)-N4-[(S)-1-(Amino-imino-methyl)-piperidin-3-ylmethyl]-2-tert-butoxycarbonylamino-N1-cyclopropyl-N1-(2-pyrazin-2-ylcarbonylamino)-succinamid-acetat (1:2) löst man in 2 ml Dioxan und versetzt diese Lösung mit 5 ml 4N Salzsäure (in Dioxan). Man rührt 2 Stunden und dampft dann das Dioxan ab. Den Rückstand legt man in 12 ml Methylenchlorid vor und versetzt unter Rühren mit 76 mg Pyrimidin-2-thioessigsäure, 0.46 ml Hünig's Base und 206 mg BOP. Das Gemisch rührt man bei Raumtemperatur und engt dann ein. Den Rückstand chromatographiert man über Kieselgel mit Essigester-Aceton-Wasser-Eisessig 4:2:1:1. Man erhält 167 mg (S)-N4-[(S)-1-(Amino-imino-methyl)-piperidin-3-ylmethyl]-N1-cyclopropyl-N1-(2-pyrazin-2-ylcarbonylamino-ethyl)-2-pyrimidin-2-ylsulfanylacetylamino-succinamid-acetat (1:2), MS (ISP): 612.5 (M + H).

Beispiel 21

Analog Beispiel 20 werden die Verbindungen

a) (S)-N4-[(S)-1-(Amino-imino-methyl)-piperidin-3-ylmethyl]-N1-cyclopropyl-2-(3-indol-3-yl-propionylamino)-N1-(2-pyrazin-2-ylcarbonylamino-ethyl)-succinamid-acetat (1:2), MS (ISP): 631.5 (M + H)

b) (S)-4-[(S)-2-[(S)-1-(Amino-imino-methyl)-piperidin-3-ylmethylcarbonyl]-1-[cyclopropyl-(2-pyrazin-2-ylcarbonylamino-ethyl)-carbamoyl]-ethylcarbamoyl]-4-hydroxy-buttersäuremethylester-acetat (1:2), MS (ISP): 604.5 (M + H)

c) (S)-2-[(S)-2-Acetylamino-3-indol-3-ylpropionylamino]-N4-[(S)-1-(amino-imino-methyl)-piperidin-3-ylmethyl]-N1-cyclopropyl-N1-(2-pyrazin-2-ylcarbonylamino-ethyl)-succinamid-acetat (1:2), MS (ISP): 688.6 (M + H)

d) (S)-N4-[(S)-1-(Amino-imino-methyl)-piperidin-3-ylmethyl]-N1-cyclopropyl-2-phenoxyacetylamino-N1-(2-pyrazin-2-ylcarbonylamino-ethyl)-succinamid-acetat (1:2), MS (ISP): 594.5 (M + H)

unter Verwendung der entsprechenden Carbonsäure anstelle von Pyrimidinthioessigsäure hergestellt.

Beispiel 22

354 mg (S)-3-[(S)-3-[Cyclopropyl-(2-pyrazin-2-ylcarbonylamino-ethyl)-carbamoyl]-3-(naphthalen-2-ylsulfonylamino)-propionyiaminomethyl]-piperidin-1-carbonsäuretert-butylester

-(hergestellt aus 2-Cyclopropylamin-ethylcarbaminsäurebenzylester-hydrochlorid via (S)-3-[(S)-3-[(2-Amino-ethyl)-cyclopropyl-carbamoyl]-3-(naphthalen-2-ylsulfonamino)-propionylaminomethyl]-piperidin-1-carbonsäure-tert-butylester-hydrochlorid)-

löst man in 3 ml Methylenchlorid und versetzt diese Lösung mit 5 ml 5N Salzsäure (in Dioxan). Nach 1 Stunde Rühren engt man diese Lösung ein und azeotropiert den Rückstand mit Wasser und dann mit Essigester. Den Rückstand rührt man in 3 ml Methylenchlorid und man gibt unter Eiskühlung 53 mg Bromcyan zu und anschliessend tropft man 0.14 ml Triäthylamin in 1 ml Methylenchlorid zu. Nach Rühren bei Raumtemperatur gibt man 70 mg Hydroxylamin-hydrochlorid und nochmals 0.14 ml Triäthylamin zu. Das Gemisch wird 20 Stunden gerührt, dann eingeengt und über Kieselgel mit Essigester/Aceton/Wasser/Eisessig 6:2:1:1 säulechromatographiert. Man erhält 60 mg (S)-N4-[(S)-1-(Amino-hydroxyimino-methyl)-piperidin-3-ylmethyl]-N1-cyclopropyl-2-(naphthalen-2-ylsulfonylamino)-N1-[2-(pyrazin-2-ylcarbonylamino)-ethyl]-succinamid-acetat (1:1), MS (ISP): 666.3 (M + H).

Beispiel 23

1.0 g (S)-N1-[3-(Benzyloxy-carbonylamino)-ethyl]-N1-cyclopropyl-2-(morpholino-sulfonyl)-amino-succinamsäurebenzylester löst man in 10 ml THF und man gibt 3.4 ml 1N LiOH-Lösung zu. Nach 1 Stunde Rühren versetzt man die Lösung mit 3.4 ml 1N Salzsäure. Die Lösung wird anschliessend in Essigester aufgenommen und mit Wasser gewaschen. Nach Eindampfen des Essigesters erhält man 997 mg freie Säure, die anschliessend gelöst in 10 ml Methylenchlorid mit 1 ml N-Ethylmorpholin, 360 mg (S)-1-Amidino-3-(aminomethyl)piperidin-dihydrochlorid und 715 mg BOP versetzt wird. Das Gemisch wird bei Raumtemperatur gerührt und anschliessend eingeengt. Den Rückstand chromatographiert man über Kieselgel mit Essigester/Aceton/Wasser/Eisessig 6:2:1:1, und es wird 740 mg 2-[[(S)-3-[(S)-1-(Amino-imino-methyl)-piperidin-3-ylmethylcarbamoyl]-2-morpholin-4-ylsulfonylamino-propionyl]-cyclopropyl-amino]-ethylcarbaminsäurebenzylester, MS (ISP): 637.5 (M+H) erhalten.

Herstellung des Ausgangsmaterials:

5.0 g 2-Cyclopropylamino-ethylcarbaminsäurebenzylester-hydrochlorid (1:1), 5.97 g BOC-Asp(OBzl)-OH und 7.16 g Hünig's Base legt man in 120 ml Methylenchlorid vor und man versetzt diese Lösung mit 8.57 g BOP. Nach 2 Stunden Rühren bei Raumtemperatur nimmt man die Lösung in Aether auf und wäscht die Aetherphasen mit 1N Salzsäure, Wasser, Bicarbonatlösung und nochmals mit Wasser. Der Eindampfrückstand wird über Kieselgel mit Essigester/Hexan 1:2 chromatographiert. Von den 9.6 g Zwischenprodukt, das man aus der Chromatographie erhält, löst man 1.76 g in 19 ml THF und versetzt diese Lösung mit 6 ml 4N Salzsäure (in Dioxan). Nach Rühren bei Raumtemperatur dampft man das Gemisch ein. Von den 2.29 g des erhaltenen Oels löst man 1.54 g in 18 ml Methylenchlorid und gibt 1.2 g Morpholin-N-sulfochlorid und 2.2 ml Hünig's Base zu. Das Gemisch wird 20 Stunden gerührt, in Essigester aufgenommen und mit 1N Salzsäure und Wasser gewaschen. Nach Trocknen und Eindampfen wird der Rückstand über Kieselgel mit Essigester/Hexan 1:1 säulechromatographiert. Man erhält 920 mg (S)-N1-[3-(Benzyloxy-carbonylamino)-ethyl]-N1-cyclopropyl-2-(morpholinosulfonyl)-amino-succinamsäurebenzylester, MS (FAB): 589.1 (M+H).

Beispiel 24

325 mg 2-[[(S)-3-[(S)-1-(Amino-imino-methyl)-piperidin-3-ylmethyl-carbamoyl]-2-morpholin-4-ylsulfonylamino-propionyl]-cyclopropyl-amino]-ethylcarbaminsäurebenzylester löst man in 10 ml Methanol. Nach Zugabe von 35 mg Pd/C (10%) hydriert man bei Raumtemperatur und anschliessend filtriert man den Katalysator ab. Nach dem Eindampfen löst man den Rückstand in 4 ml Methylenchlorid und gibt unter Rühren 48 mg Pyrazincarbonsäure, 0.24 ml N-Ethylmorpholin und 178 mg BOP zu. Nach Rühren bei Raumtemperatur engt man das Gemisch ein und chromatographiert den Rückstand über Kieselgel mit Essigester/Aceton/Wasser/Eisessig 6:2:1:1. Man erhält 181 mg (S)-N4-[(S)-1-(Amino-imino-methyl)-piperidin-3-ylmethyl]-N1-cyclopropyl-2-morpholin-4-ylsulfonylamino-N1-(2-pyrazin-2-ylcarbonylamino-ethyl)-succinamid-acetat (1:1), MS (ISP): 609.5 (M+H).

Beispiel 25

Wird an Stelle der im Beispiel 24 eingesetzten Pyrazincarbonsäure, die 2,3-Dihydro-3-oxopyridazin-6-carbonsäure eingesetzt, erhält man

das (S)-N4-[(S)-1-(Amino-imino-methyl)-piperidin-3-ylmethyl]-N1-cyclopropyl-2-morpholin-4-ylsulfonylamino-N1-[2-(6-oxo-1,6-dihydro-pyridazin-3-ylcarbonylamino-ethyl]-succinamid-acetat (1:1), MS (ISP): 625.4 (M+H).

Beispiel 26

Werden an Stelle der im Beispiel 24 eingesetzten Pyrazincarbonsäure die folgenden Säuren:

a) 6-Oxo-1,4,5,6-tetrahydro-pyridazincarbonsäure,
b) 3-Amino-pyrazin-2-carbonsäure,
c) Methoxy-essigsäure,
d) Morpholino-essigsäure,
e) 4-Methyl-piperazin-1-essigsäure,
f) 3-Methoxy-propionsäure,
g) 1-Imidazolyl-essigsäure bzw.
h) 2-Hydroxyamino-glutarsäure

eingesetzt, so erhält man folgende Produkte:

a) (S)-N4-[(S)-1-(Amino-imino-methyl)-piperidin-3-ylmethyl]-N1-cyclo-propyl-2-morpholin-4-ylsulfonylamino-N1-[2-(6-oxo-1,4,5,6-tetrahydro-pyridazin-3-ylcarbonylamino)-ethyl]-succinamid-acetat (1:1), MS (ISP): 627,6 (M+H),
b) (S)-N4-[(S)-1-(Amino-imino-methyl)-piperidin-3-ylmethyl]-N1-[2-(3-amino-pyrazin-2-ylcarbonylamino)-ethyl]-N1-cyclopropyl-2-morpholin-4-ylsulfonylamino-succinamid-acetat (1:1), MS (ISP): 624,6 (M+H),
c) (S)-N4-[(S)-1-(Amino-imino-methyl)-piperidin-3-ylmethyl]-N1-cyclopropyl-N1-(2-methoxyacetylamino-ethyl)-2-morpholin-4-ylsulfonylamino-succinamid-acetat (1:1), MS (ISP): 575,5 (M+H),

d) (S)-N4-[(S)-1-(Amino-imino-methyl)-piperidin-3-ylmethyl]-N-1-cyclo-propyl-N1-(2-morpholin-4-ylacetylamino-ethyl)-2-morpholin-4-ylsulfonyl-amino-succinamid-acetat (1:1), MS (ISP): 630,6 (M+H) und 316,2 (M+2H),

e) (S)-N4-[(S)-1-(Amino-imino-methyl)-piperidin-3-ylmethyl]-N1-cyclo-propyl-N1-[2-(4-methyl-piperazin-1-ylacetylamino)-ethyl]-2-morpholin-4-ylsulfonylamino-succinamid-acetat (1:1), MS (ISP): 643,6 (M+H),

f) (S)-N4-[(S)-1-(Amino-imino-methyl)-piperidin-3-ylmethyl-N1-cyclo-propyl-N1-[2-(3-methoxy-propionylamino)-ethyl]-2-morpholin-4-ylsulfonyl-amino-succinamid-acetat (1:1), MS (ISP): 589,6 (M+H),

g) (S)-N4-[(S)-1-(Amino-imino-methyl)-piperidin-3-ylmethyl]-N1-cyclopropyl-N1-(2-imidazol-1-ylacetylamino-ethyl)-2-morpholin-4-ylsulfonyl-amino-succinamid-acetat (1:1), MS (ISP): 611,6 (M),

h) (E)- und (Z)-4-[2-[[(S)-3-[(S)-1-(Amino-imino-methyl)-piperidin-3-ylmethylcarbamoyl]-2-morpholin-4-ylsulfonylaminopropionyl]-cyclopropyl-amino]-ethylcarbamoyl]-4-hydroxyimino-buttersäure-acetat (1:1), MS (ISP): 646,5 (M+H).

Beispiel 27

2,77 g 2-[[(S)-3-[(S)-1-(Amino-imino-methyl)-piperidin-3-ylmethyl-carbamoyl]-2-morpholin-4-ylsulfonylamino-propionyl]-cyclopropyl-amino]-ethylcarbaminsäurebenzylester (Beispiel 23) löst man in 90 ml Methanol. Nach Zugabe von 2,13 ml 2N Salzsäure und 300 mg Pd/C (10%) hydriert man 1,5 Stunden bei Raumtemperatur, filtriert dann den Katalysator ab und engt das Filtrat ein. Den Rückstand dispergiert man mit Aether und filtriert die weissen Kristalle ab. Man erhält 2,44 g kristallines (S)-N1-(2-Amino-ethyl)-N4-[(S)-1-(amino-imino-methyl)-piperidin-3-ylmethyl]-N1-cyclopropyl-2-morpholin-4-ylsulfonylamino-succinamid-hydrochlorid (1:2), MS (ISP): 503,4 (M+H).

Beispiel 28

330 mg (S)-N1-(2-Amino-ethyl)-N4-[(S)-1-(amino-imino-methyl)-piperidin-3-ylmethyl]-N1-cyclopropyl-2-morpholin-4-ylsulfonylamino-succinamid-hydrochlorid rührt man zusammen mit 162 mg 4-Methyl-piperazin-1-yl-sulfonylchlorid und 0,49 ml Hünig's Base in 8 ml Methylenchlorid 20 Stunden bei Raumtemperatur. Das Reaktionsgemisch chromatographiert man über Kieselgel mit Essigester/Aceton/Eisessig/Wasser 2:2:2:1. Man erhält 149,1 mg reines kristallines (S)-N4-[(S)-1-(Amino-imino-methyl)-piperidin-3-ylmethyl]-N1-cyclopropyl-N1-[2-(4-methyl-piperazin-1-ylsulfonylamino)-ethyl]-2-morpholin-4-ylsulfonylamino-succinamid-acetat (1:2), MS (ISP): 665,6 (M+H) und 333,7 (M+2H).

Beispiel 29

Analog Beispiel 28 erhält man unter Verwendung von Morpholino-sulfonylchlorid an Stelle von 4-Methyl-piperazin-1-ylsulfonylchlorid das (S)-N4-[(S)-1-(Amino-imino-methyl)-piperidin-3-ylmethyl-N1-cyclopropyl-2-morpholin-4-ylsulfonylamino-N1-(2-morpholin-4-ylsulfonylamino-ethyl)-succinamid-acetat (1:1), MS (ISP): 652,5 (M+H).

Beispiel 30

Analog Beispiel 20 jedoch unter Verwendung der Carbonsäure:

a) 3-Methoxy-propionsäure,
b) Morpholinoessigsäure,
c) Methoxyessigsäure,
d) 4-Methyl-piperazin-1-essigsäure,
e) (L)-Phenylalanin,
f) (D)-Phenylalanin,
g) 4-Hydroxy-piperidin-1-essigsäure,
h) 1-Imidazolyl-essigsäure,
i) 2-Piperidon-1-essigsäure,
j) 3-Dimethylamino-propionsäure,
k) Dimethylamino-essigsäure bzw.
l) 2,6-Dimethylmorpholin-4-essigsäure.

an Stelle von Pyrimidinthioessigsäure werden folgende Verbindungen hergestellt:

a) (S)-N4-[(S)-1-(Amino-imino-methyl)-piperidin-3-ylmethyl]-N1-cyclo-propyl-2-(3-methoxy-propionylamino)-N1-(2-pyrazin-2-ylcarbonylamino-ethyl)-succinamid-acetat (1:2), MS (ISP): 546,5 (M+H),

b) (S)-N4-[(S)-1-(Amino-imino-methyl)-piperidin-3-ylmethyl]-N1-cyclo-propyl-2-morpholin-4-ylacetylamino-N1-(2-pyrazin-2-ylcarbonylamino-ethyl)-succinamid-acetat (1:3), MS (EI): 587,3 (M+H),

c) (S)-N4-[(S)-1-(Amino-imino-methyl)-piperidin-3-ylmethyl]-N1-cyclo-propyl-2-methoxyacetylamino-N1-(2-pyrazin-2-ylcarbonylamino-ethyl)-succinamid-acetat (1:2), MS (ISP): 532,6 (M+H),

d) (S)-N4-[(S)-1-(Amino-imino-methyl)-piperidin-3-ylmethyl]-N1-cyclo-propyl-2-(4-methyl-piperazin-1-ylacetylamino)-N1-(2-pyrazin-2-ylcarbonyl-amino-ethyl)-succinamid-acetat (1:3), MS (ISP): 600,6 (M+H),

e) (S)-N4-[(S)-1-(Amino-imino-methyl)-piperidin-3-ylmethyl]-2-[(S)-2-amino-3-phenyl-propionylamino]-N1-cyclopropyl-N1-(2-pyrazin-2-ylcarbonyl-amino-ethyl)-succinamid-hydrochlorid (1:2), MS (ISP): 607,5 (M+H),

f) (S)-N4-[(S)-1-(Amino-imino-methyl)-piperidin-3-ylmethyl-2-[(R)-2-amino-3-phenyl-propionylamino]-N1-cyclopropyl-N1-(2-pyrazin-2-ylcarbonyl-amino-ethyl)-succinamid-hydrochlorid (1:2), MS (ISP): 607,5 (M+H) und 304,6 (M+2H),

g) (S)-N4-[(S)-1-(Amino-imino-methyl)-piperidin-3-ylmethyl]-N1-cyclo-propyl-2-[2-(4-hydroxy-piperidin-1-yl)-acetylamino]-N1-(2-pyrazin-2-ylcarbonylamino-ethyl)-succinamid-hydrochlorid (1:2), MS (ISP): 601,6 (M+H),

h) (S)-N4-[(S)-1-(Amino-imino-methyl)-piperidin-3-ylmethyl]-N1-cyclo-propyl-2-(2-imidazol-1-yl-acetylamino)-N1-(2-pyrazin-2-ylcarbonylamino-ethyl)-succinamid-hydrochlorid (1:2), MS (ISP): 568,5 (M+H),

i) (S)-N4-[(S)-1-(Amino-imino-methyl)-piperidin-3-ylmethyl]-N1-cyclo-propyl-2-[2-(2-oxo-piperidin-1-yl)-acetylamino]-N1-(2-pyrazin-2-ylcarbonyl-amino-ethyl)-succinamid-hydrochlorid (1:2), MS (ISP): 599,5 (M+H),

j) (S)-N4-[(S)-1-(Amino-imino-methyl)-piperidin-3-ylmethyl]-N1-cyclo-propyl-2-(3-dimethylamino-propionylamino)-N1-(2-pyrazin-2-ylcarbonyl-amino-ethyl)-succinamid-hydrochlorid (1:3), MS (ISP): 559,6 (M+H),

k) (S)-N4-[(S)-1-(Amino-imino-methyl)-piperidin-3-ylmethyl]-N1-cyclo-propyl-2-(2-dimethylamino-acetylamino)-N1-(2-pyrazin-2-ylcarbonylamino-ethyl)-succinamid-hydrochlorid (1:3), MS (ISP): 545,6 (M+H),

l) (S)-N4-[(S)-1-(Amino-imino-methyl)-piperidin-3-ylmethyl]-N1-cyclo-propyl-2-(2,6-dimethyl-morpholin-4-ylacetylamino)-N1-(2-pyrazin-2-ylcarbonylamino-ethyl)-succinamid-hydrochlorid (1:2) (R:S=1:1 für C2 und C6 im Morpholinoring), MS: 615,4 (M+H).

Beispiel 31

Eine Lösung von 3,0 g des Produkts von Beispiel 7.B)a) in 63 ml Ethanol und 7 ml Wasser werden mit 0,95 ml Zimtaldehyd und 1,6 ml 4-Ethyl-morpholin versetzt und 20 Minuten bei Raumtemperatur gerührt. Zu dieser Lösung gibt man 0,3 g Pd/C-Katalysator und hydriert das Reaktionsgemisch während 6 Stunden bei Normalbedingungen. Dann wird der Katalysator abfiltriert, das Filtrat eingedampft und der Rückstand mit einem Wasser-Acetonitril Gradienten an RP-18 chromatographiert. Die Produktfraktionen werden eingedampft und mit Wasser-Ethanol über einen Ionentauscher ($Cl^-$-Form) filtriert. Nach Eindampfen isoliert man farbloses 3-[[(S)-3-[(S)-1-(Amino-imino-methyl)-piperidin-3-ylmethylcarbamoyl]-2-(3-phenyl-propyl-amino)-propionyl]-cyclopropyl-amino]-propionsäureethylester-hydrochlorid (1:2), MS (Ion-Spray): 529,6 $(M+H)^+$.

Beispiel 32

Analog Beispiel 31 stellt man folgendes Produkt her:

3-[[(S)-3-[(S)-1-(Amino-imino-methyl)-piperidin-3-ylmethylcarbamoyl]-2-butylamino-propionyl]-cyclopropyl-amino]-propionsäureethylester-hydrochlorid (1:2), MS (Ion-Spray): 467,6 $(M+H)^+$.

Beispiel 33

Analog Beispiel 3, aber mit einer zusätzlichen Filtration des Endproduktes mit Wasser-Ethanol über einen Ionenaustauscher ($Cl^-$-Form) erhält man folgende Produkte:

a) 3-[[(S)-3-[(S)-1-(Amino-imino-methyl)-piperidin-3-ylmethylcarbamoyl]-2-(3-phenyl-propylamino)-propionyl]-cyclopropyl-amino]-propionsäure-hydrochlorid (1:1), MS (Ion-Spray): 501,7 $(M+H)^+$,

b) 3-[[(S)-3-[(S)-1-(Amino-imino-methyl)-piperidin-3-ylmethylcarbamoyl]-2-butylamino-propionyl]-cyclopropyl-amino]-propionsäure-hydrochlorid (1:1), MS (Ion-Spray): 439,6 $(M+H)^+$.

Beispiel 34

Zu einer Lösung von 3,4 g des Produktes von Beispiel 3 in 34 ml Ethanol und 6,8 ml 1N Salzsäure gibt man 0,34 g Palladium/Kohle Katalysator und hydriert das Reaktionsgemisch während 2 Stunden. Dann wird

der Katalysator abfiltriert und das Filtrat eingedampft. Man isoliert so farbloses [[(S)-2-Amino-3-[(S)-1-(amino-imino-methyl)-piperidin-3-ylmethylcarbamoyl]-propionyl]-cyclopropyl-amino]-essigsäure-hydrochlorid (1:1), MS (Ion-Spray): 369,4 $(M+H)^+$.

Beispiel 35

Analog Beispiel 34 stellt man folgende Verbindung aus dem Produkt von Beispiel 37 her:

(S)-3-Amino-N-[(S)-1-(amino-imino-methyl)-piperidin-3-ylmethyl]-4-azepan-1-yl-4-oxo-butyramid-hydrochlorid (1:2), MS (Ion-Spray): 353,4 $(M+H)^+$.

Beispiel 36

2,1 g des Produktes von Beispiel 7.A) werden in 45 ml Ethanol und 5 ml Wasser gelöst, mit 0,7 g Zimtaldehyd und 1,1 ml 4-Ethylmorpholin versetzt und 15 Minuten bei Raumtemperatur gerührt. Dann gibt man 0,2 g Pd/C Katalysator zu und hydriert 6 Stunden bei Normalbedingungen. Man filtriert den Katalysator ab, dampft das Filtrat ein und chromatographiert den Rückstand mit einem Wasser-Acetonitril Gradienten an RP-18. Die Produktfraktionen werden eingedampft und der Rückstand über einen Ionenaustauscher (Dowex, $Cl^-$-Form) filtriert. Man isoliert nach Eindampfen 0,5 g N-[(S)-1-(Amino-imino-methyl)-piperidin-3-ylmethyl]-2-[(S)-4-cyclopropyl-3,6-dioxo-1-(3-phenyl-propyl)-piperazin-2-yl]-acetamid-hydrochlorid (1:1), MS (ISP): 469,5 $(M+H)^+$.

Beispiel 37

Analog Beispiel 1 stellt man folgende Verbindung her:

(S)-N-[(S)-1-(Amino-imino-methyl)-piperidin-3-ylmethyl]-4-azepan-1-yl-3-benzyloxycarbonylamino-4-oxo-butyramid-hydrochlorid (1:1), MS (Ion-Spray): 487,5 $(M+H)^+$.

Herstellung des Ausgangsmaterials:

Analog Beispiel 1.B), jedoch unter Verwendung von Hexamethylenimin anstelle von N-Cyclopropylglycinethylester stellt man folgendes Zwischenprodukt her:

(S)-4-Azepan-1-yl-3-benzyloxycarbonylamino-4-oxo-buttersäure-t-butyl-ester, MS (Ion-Spray): 405,2 $(M+H)^+$.

Beispiel 38

Analog Beispiel 9 jedoch unter Verwendung der entsprechenden aktivierten Carbamate werden folgende Verbindungen hergestellt:

a) [[(S)-3-[(S)-1-(Amino-imino-methyl)-piperidin-3-ylmethylcarbamoyl]-2-(3-diphenylmethyl-ureido)-propionyl]-cyclopropyl-amino]-essigsäureethylester-hydrochlorid (1:1), MS (Ion-Spray): 606,6 $(M+H)^+$,

b) [[(S)-3-[(S)-1-(Amino-imino-methyl)-piperidin-3-ylmethylcarbamoyl]-2-(3-cyclohexylmethyl-ureido)-propionyl]-cyclopropyl-amino]-essigsäureethylester-hydrochlorid (1:1), MS (Ion-Spray): 536,5 $(M+H)^+$.

Herstellung der Ausgangsmaterialien:

Analog Beispiel 9.B)b) werden folgende aktivierte Carbamate hergestellt:

Diphenylmethyl-carbaminsäure-2,5-dioxo-pyrrolidin-1-ylester, MS (FAB): 209 (M-Hydroxysuccinimid),

Cyclohexylmethylcarbaminsäure-2,5-dioxo-pyrrolidin-1-ylester, MS (FAB): 115 (M-Hydroxysuccinimid).

Beispiel 39

Analog Beispiel 9 jedoch ausgehend von dem Amin aus Beispiel 35 und unter Verwendung der entsprechenden aktivierten Carbamate werden folgende Verbindungen hergestellt:

a) (S)-N-[(S)-1-(Amino-imino-methyl)-piperidin-3-ylmethyl]-4-azepan-1-yl-3-(3-benzyl-ureido)-4-oxo-butyramid-hydrochlorid (1:1), MS (Ion-Spray): 486,4 $(M+H)^+$,

b) (S)-2-[3-[(S)-1-[(S)-(Amino-imino-methyl)-piperidin-3-ylmethylcarbamoylmethyl]-2-azepan-1-yl-2-oxo-ethyl]-ureido]-3-phenylpropionsäuremethylester-hydrochlorid (1:1), MS (Ion-Spray): 558,5 $(M+H)^+$,

c) (R)-2-[3-[(S)-1-[(S)-(Amino-imino-methyl)-piperidin-3-ylmethylcarbamoylmethyl]-2-azepan-1-yl-2-oxo-ethyl]-ureido]-3-phenylpropionsäuremethylester-hydrochlorid (1:1), MS (Ion-Spray): 558,5 $(M+H)^+$.

Herstellung der Ausgangsmaterialien:

Analog Beispiel 9.B)b) werden folgende aktivierte Carbamate hergestellt:

Benzylcarbaminsäure-2,5-dioxo-pyrrolidin-1-ylester, MS (FAB): 133 (M-Hydroxysuccinimid),

(R)-2-(2,5-Dioxo-pyrrolidin-1-yloxycarbonylamino)-3-phenylpropionsäuremethylester, MS (FAB): 133 (M-Hydroxysuccinimid),

(S)-2-(2,5-Dioxo-pyrrolidin-1-yloxycarbonylamino)-3-phenylpropionsäuremethylester, MS (FAB): 162 (M-$H_2$NCOO-succinimid).

Beispiel 40

Analog Beispiel 3 jedoch unter Verwendung der Produkte von Beispiel 38 und 39 b) und c) werden folgende Produkte hergestellt:

a) [[(S)-3-[(S)-1-(Amino-imino-methyl)-piperidin-3-ylmethylcarbamoyl]-2-(3-diphenylmethyl-ureido)-propionyl]-cyclopropyl-amino]-essigsäure, MS (Ion-Spray): 578,3 $(M+H)^+$,

b) [[(S)-3-[(S)-1-(Amino-imino-methyl)-piperidin-3-ylmethylcarbamoyl]-2-(3-cyclohexylmethyl-ureido)-propionyl]-cyclopropyl-amino]-essigsäure, MS (Ion-Spray): 508,5 $(M+H)^+$,

c) (S)-2-[3-[(S)-1-[(S)-(Amino-imino-methyl)-piperidin-3-ylmethylcarbamoylmethyl]-2-azepan-1-yl-2-oxo-ethyl]-ureido]-3-phenylpropionsäure, MS (Ion-Spray): 544,5 $(M+H)^+$,

d) (R)-2-[3-[(S)-1-[(S)-(Amino-imino-methyl)-piperidin-3-ylmethylcarbamoylmethyl]-2-azepan-1-yl-2-oxo-ethyl]-ureido]-3-phenylpropionsäure, MS (Ion-Spray): 544,6 $(M+H)^+$.

Beispiel 41

Analog Beispiel 1 jedoch unter Verwendung von (S)-(3-Aminomethyl-piperidin-1-yl)-imino-methylcarbaminsäureethylester-hydrochlorid an Stelle von (S)-1-Amidino-3-(aminomethyl)piperidin-dihydrochlorid erhält man die [[(S)-2-Benzyloxycarbonylamino-3-[(S)-1-(ethoxycarbonylamino-imino-methyl)-piperidin-3-ylmethylcarbamoyl]-propionyl]-cyclopropyl-amino]-essigsäure, MS (Ion-Spray): 575,5 $(M+H)^+$.

Beispiel 42

Analog Beispiel 41 jedoch unter Verwendung von (RS)-(2-Amino-methyl-morpholin-4-yl)-imino-methylcarbaminsäureethylester-hydrochlorid an Stelle von (S)-(3-Aminomethyl-piperidin-1-yl)-imino-methylcarbaminsäureethylester-hydrochlorid erhält man die [[(S)-2-Benzyloxycarbonyl-amino-3-[4-(ethoxycarbonylamino-imino-methyl)-morpholin-2-ylmethyl-carbamoyl]-propionyl]-cyclopropyl-amino]-essigsäure (R:S = 1:1 im Morpholinring), MS (Ion-Spray): 577,5 $(M+H)^+$.

Beispiel 43

Analog Beispiel 15 aber ausgehend von

a) [(S)-3-[(S)-1-(Amino-imino-methyl)-piperidin-3-ylmethylcarbamoyl]-2-naphthalen-2-ylsulfonylamino-propionylamino]-essigsäure bzw.

b) (S)-2-[(S)-3-[(S)-1-(Amino-imino-methyl)-piperidin-3-ylmethylcarbamoyl]-2-naphthalen-2-ylsulfonylamino-propionylamino]-3-hydroxypropionsäure

stellt man folgende Produkte her:

a) N-[(S)-1-(Amino-imino-methyl)-piperidin-3-ylmethyl]-2-[(S)-1-naphthalen-2-ylsulfonyl-3,6-dioxo-piperazin-2-yl]-acetamid-hydrochlorid (1:1), MS (Ion-Spray): 501,4 $(M+H)^+$,

b) N-[(S)-1-(Amino-imino-methyl)-piperidin-3-ylmethyl]-2-[(2S,5S)-5-hydroxymethyl-1-naphthalen-2-ylsulfonyl-3,6-dioxo-piperazin-2-yl]-acetamid-hydrochlorid (1:1), MS (Ion-Spray): 531,4 $(M+H)^+$.

Herstellung der Ausgangsmaterialien:

1. Ausgehend von N-Boc-L-Asparaginsäure-β-t-butylester und von Glycinethylester bzw. Serinmethylester erhält man via

1.a) t-Butyl-(S)-3-(1-t-butoxyformamido)-N-[(ethoxycarbonyl)methyl]-succinamat bzw.

1.b) t-Butyl-(S)-3-(1-t-butoxyformamido)-N-[1-(2-hydroxy-1-methoxycarbonyl)-ethyl]succinamat, via

2.a) t-Butyl-(S)-N-[(ethoxycarbonyl)methyl]-3-(naphthalen-2-ylsulfonamido)-succinamat bzw.

2.b) t-Butyl-(S)-N-[1-(2-hydroxy-1-methoxycarbonyl)-ethyl]-3-(naphthalen-2-ylsulfonamido)succinamat und via

3.a) [(S)-3-[(S)-1-(Amino-imino-methyl)-piperidin-3-ylmethylcarbamoyl]-2-naphthalen-2-yl-sulfonylamino-propionylamino-essigsäureethylester-hydrochlorid (1:1), MS (ISP): 547,4 $(M+H)^+$ bzw.

3.b) (S)-2-[(S)-3-[(S)-1-(Amino-imino-methyl)-piperidin-3-ylmethylcarbamoyl]-2-naphthalen-2-ylsulfonylamino-propionylamino]-3-hydroxy-propionsäuremethylester-hydrochlorid (1:1), MS (ISP): 563,4 $(M+H)^+$ die

4.a) [(S)-3-[(S)-1-(Amino-imino-methyl)-piperidin-3-ylmethylcarbamoyl]-2-naphthalen-2-ylsulfonylamino-propionylamino]-essigsäure, MS (ISP): 519,4 $(M+H)^+$ bzw. die

4.b) (S)-2-[(S)-3-[(S)-1-(Amino-imino-methyl)-piperidin-3-ylmethylcarbamoyl]-2-naphthalen-2-ylsulfonylamino-propionylamino]-3-hydroxypropionsäure, MS (ISP): 549,3 $(M+H)^+$.

Beispiel 44

Analog Beispiel 1.A) erhält man aus einem (1:1)-Gemisch von (2S,5R)-und (2S,5S)-(5-Benzyl-3,6-dioxo-4-propyl-piperazin-2-yl)-essigsäure-t-butylester ein (1:1)-Gemisch von

(2S,5R) und (2S,5S)-(5-Benzyl-3,6-dioxo-4-propyl-piperazin-2-yl)-essigsäure (S)-1-(amino-imino-methyl)-piperidin-3-ylmethylamid-hydrochlorid (1:1), MS (Ion-Spray): 443,6 $(M+H)^+$.

Herstellung des Ausgangsmaterials:

Ausgehend von N-Boc-L-Asparaginsäure-$\beta$-t-butylester und rac-N-Propyl-phenylalanin-methylester erhält man via (S)-2-t-Butoxycarbonyl-amino-N-[(R)- und [(S)-1-methoxycarbonyl-2-phenyl-ethyl]-N-propylsuccinamsäure-t-butylester, MS (ISP): 493,5 $(M+H)^+$ den (2S,5R)- und (2S,5S)-(5-Benzyl-3,6-dioxo-4-propyl-piperazin-2-yl)-essigsäure-t-butylester, MS (FAB): 360 $(M^+)$.

Beispiel 45

Analog Beispiel 1.A) erhält man aus (S)-(4-Cyclopropyl-1-ethoxy-carbonylmethyl-3,6-dioxo-piperazin-2-yl)-essigsäure-t-butylester das (S)-[2-[(S)-1-(Amino-imino-methyl)-piperidin-3-ylmethylcarbamoylmethyl]-4-cyclopropyl-3,6-dioxo-piperazin-1-yl]-essigsäure-ethylester-hydrochlorid (1;1), MS (Ion-Spray): 437,5 $(M+H)^+$.

Herstellung des Ausgangsmaterials:

a) 0,67 g des Produktes von Beispiel 7.A) werden in 7 ml DMF gelöst, mit 0,5 ml 4-Ethylmorpholin versetzt und über Nacht bei 80 °C gerührt. Das Reaktionsgemisch wird eingedampft und der Rückstand mit Essigester-Methanol (99:1) an Kieselgel chromatographiert. Man erhält so 0,43 g farbloses (S)-(4-Cyclopropyl-3,6-dioxo-piperazin-2-yl)-essigsäure-t-butylester, MS (Ion-Spray): 269,3 $(M+H)^+$.

b) Zu einer Lösung von 3,45 g des unter a) erhaltenen Produktes in 50 ml DMF gibt man 1,9 g Natriumhydrid-Dispersion (80%ig) und tropft anschliessend bei 3-10 °C 7,1 ml Bromessigsäureethylester zu. Man rührt 4 Stunden bei Raumtemperatur, giesst dann das Reaktionsgemisch auf Wasser und extrahiert mit Essigester. Die organische Phase wird mit Kochsalzlösung gewaschen, getrocknet und eingedampft. Nach Chromatographie des Rückstandes mit Essigester-Hexan (1:1) an Kieselgel erhält man 1,6 g (S)-(4-Cyclopropyl-1-ethoxycarbonylmethyl-3,6-dioxo-piperazin-2-yl)-essigsäure-t-butylester in Form eines gelben Harzes, MS (Ion-Spray): 355,4 $(M+H)^+$.

Beispiel 46

Analog Beispiel 3 erhält man aus dem Produkt von Beispiel 45 die

[(S)-2-[(S)-1-(Amino-imino-methyl)-piperidin-3-ylmethylcarbamoyl-methyl]-4-cyclopropyl-3,6-dioxo-piperazin-1-yl]-essigsäure, MS (Ion-Spray): 409,5 $(M+H)^+$.

Beispiel 47

Eine Lösung von 6,5 g (RS)-(2,4-Dimethyl-1-naphthalen-2-ylsulfonyl-3-oxo-piperazin-2-yl)-essigsäure in 65 ml DMF wird nacheinander mit 7,64 g BOP, 6,5 ml 4-Ethylmorpholin und 4,35 g (S)-1-Amidino-3-(aminomethyl)-piperidin-dihydrochlorid versetzt und über Nacht bei Raumtemperatur gerührt. Nach Eindampfen des Lösungsmittels wird der Rückstand mit einem Wasser-Acetonitril Gradienten an RP-18 chromatographiert. Die Produktfraktionen werden eingedampft und der Rückstand über einen Ionenaustauscher (Dowex, $Cl^-$-Form) filtriert. Man isoliert nach Eindampfen 0,8 g eines (1:1)-Gemisches von (R)- und (S)-(2,4-Dimethyl-1-naphthalen-2-ylsulfonyl-3-oxo-piperazin-2-yl)-essigsäure-(S)-1-(amino-imino-methyl)-piperidin-3-ylmethylamid-hydrochlorid (1:1), MS (Ion-Spray): 515,6 $(M+H)^+$.

Herstellung des Ausgangsmaterials:

a) Eine Lösung von 10 g Ethyl-2-pyrazin-3-on-acetat in 100 ml Dioxan wird mit 6,8 ml 4-Ethylmorpholin und einer Lösung von 12,9 g Di-tert-butyldicarbonat in 50 ml Dioxan versetzt und 6 Stunden bei

Raumtemperatur gerührt. Das Lösungsmittel wird abgedampft und der Rückstand mit Essigester-Hexan (1:1) an Kieselgel gereinigt. Man erhält 15,4 g (RS)-2-Ethoxycarbonyl-methyl-3-oxo-piperazin-1-carbonsäure-t-butylester, MS (Ion-Spray): 287,3 $(M+H)^+$.

b) Eine Lösung von 15,0 g der unter a) erhaltenen Verbindung in 200 ml DMF wird mit 7,9 g Natriumhydrid-Dispersion (80%ig) versetzt. Anschliessend tropft man bei 3-10 °C 16,3 ml Methyljodid zu, rührt 4 Stunden bei Raumtemperatur, giesst dann das Reaktionsgemisch auf Wasser und extrahiert mit Essigester. Die organische Phase wird mit Wasser gewaschen, getrocknet und eingedampft. Der Rückstand wird mit Essigester Hexan (1:1) an Kieselgel chromatographiert. Man erhält 11,4 g (RS)-2-Ethoxycarbonylmethyl-2,4-dimethyl-3-oxo-piperazin-1-carbonsäure-t-butylester, MS (FAB): 314 $(M^+)$.

c) Eine Lösung des Produkts von b) in Acetonitril wird mit p-Toluolsulfonsäure-monohydrat versetzt. Nach Eindampfen wird eine Lösung des Rückstandes in Dioxan mit β-Naphthylsulfochlorid versetzt. Man erhält den (RS)-(2,4-Dimethyl-1-naphthalen-2-ylsulfonyl-3-oxo-piperazin-2-yl)-essigsäureethylester, MS (FAB): 405 $(M+H)^+$.

d) Analog Beispiel 3 erhält man aus dem Produkt von c) die (RS)-(2,4-Dimethyl-1-naphthalen-2-ylsulfonyl-3-oxo-piperazin-2-yl)-essigsäure, MS (Ion-Spray): 375,4 $(M-H)^-$.

Beispiel 48

2,35 g des Aminprodukts von Beispiel 7.A) werden in 25 ml DMF mit 3,2 ml 4-Ethylmorpholin und 0,5 ml Chloressigsäurechlorid versetzt und über Nacht bei Raumtemperatur gerührt. Das Reaktionsgemisch wird eingedampft und der Rückstand an einer RP-18 Säule mit einem Wasser-Acetonitril Gradienten chromatographiert. Aus den Produktfraktionen erhält man nach Eindampfen 0,5 g [[(S)-3-[(S)-1-Amino-imino-methyl)-piperidin-3-ylmethylcarbamoyl]-2-(2-chlor-acetylamino)-propionyl]-cyclopropyl-amino]-essigsäureethylester-hydrochlorid (1:1), MS (ISP): 473,4 $(M+H)^+$.

Beispiel 49

Analog Beispiel 3 erhält man aus dem Produkt von Beispiel 48 die [[(S)-3-[(S)-1-Amino-imino-methyl)-piperidin-3-ylmethylcarbamoyl]-2-(2-chlor-acetylamino)-propionyl]-cyclopropyl-amino]-essigsäure, MS (ISP): 445,4 $(M+H)^+$.

Beispiel 50

0,9 g des Aminprodukts aus Beispiel 7.A) werden zusammen mit 0,5 g Natriumhydrogencarbonat in 9 ml THF und 9 ml Wasser gelöst, mit 0,82 g Morpholin-N-sulfochlorid in 8 ml THF und 1,9 ml 1N Natronlauge versetzt und 48 Stunden bei Raumtemperatur gerührt. Das Reaktionsgemisch wird mit 1N Salzsäure sauer gestellt, eingedampft und der Rückstand an einer RP-18 Säule mit einem Wasser-Acetonitril Gradienten chromatographiert. Aus den Produktfraktionen erhält man nach Eindampfen 0,2 g farbloses 3-[[(S)-3-[(S)-1-(Amino-imino-methyl)-piperidin-3-ylmethylcarbamoyl]-2-morpholin-4-ylsulfonylamino-propionyl]-cyclopropyl-amino]-propionsäureethylester-hydrochlorid (1:1), MS (ISP): 560,5 $(M+H)^+$.

Beispiel 51

Analog Beispiel 3 erhält man aus dem Produkt von Beispiel 50 die 3-[[(S)-3-[(S)-1-(Amino-imino-methyl)-piperidin-3-ylmethylcarbamoyl]-2-morpholin-4-ylsulfonylamino-propionyl]-cyclopropyl-amino]-propionsäure, MS (Ion-Spray): 532,5 $(M+H)^+$.

Eine Verbindung der Formel I, ein Solvat oder Salz davon kann man in an sich bekannter Weise als Wirkstoff zur Herstellung von pharmazeutischen Präparaten, z.B. von Tabletten und Kapseln folgender Zusammensetzung, verwenden:

Beispiel A

| | pro Tablette |
|---|---|
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

Beispiel B

| | pro Kapsel |
|---|---|
| Wirkstoff | 100,0 mg |
| Maisstärke | 20,0 mg |
| Milchzucker | 95,0 mg |
| Talk | 4,5 mg |
| Magensiumstearat | 0,5 mg |
| | 220,0 mg |

**Patentansprüche**

**1.** Carboxamide der Formel

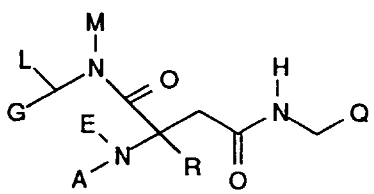

I

worin

| | |
|---|---|
| E | Wasserstoff, |
| G | H, nieder-Alkyl oder gegebenenfalls über nieder-Alkylen gebundenes COOH, COO-nieder-Alkyl, nieder-Alkanoyl, OH, nieder-Alkanoyloxy, nieder-Alkoxy, Aryl-niederalkoxy, $CONH_2$, $CONHCH_2CH_2OH$, CONHOH, $CONHOCH_3$, CONHO-Benzyl, $CONHSO_2$-nieder-Alkyl, $CONHCH_2CH_2$-Aryl, CONH-Cycloalkyl, $CONHCH_2$-Heteroaryl, $NH_2$, NHCOO-nieder-Alkyl, NHCOO-nieder-Aralkyl, $NHSO_3H$, ($NHSO_2$ oder $NHSO_3$)-nieder-Alkyl, NH-nieder-Alkanoyl, NHCOCOOH, NHCOCOO-nieder-Alkyl, NH-Cycloalkyl, NH-(3,4-Dioxo-2-hydroxy-cyclobut-1-enyl), NH-[2-nieder-(Alkoxy oder -Alkenyloxy)-3,4-dioxocyclobut-1-enyl], $NHCH_2$-Heteroaryl, NHCOCO-(Aryl oder nieder-Alkyl), $NHCOCH_2Cl$, NHCO-nieder-Alkylen-O-(niederalkyl oder aryl), $NHCOCH_2$[Aryl, Heteroaryl oder -N(Het)], NHCOC(NOH)-nieder-Alkylen-COOH, $NHSO_2$-N(Het), NHCO-(Aryl, Heteroaryl oder Heterocyclyl), $NHPO_3(R^{10},R^{20})$, Heteroaryl oder eine Gruppe CO-N(Het), |
| $R^{10}$ und $R^{20}$ | H, nieder-Alkyl oder Phenyl, und |
| M | H, nieder-Alkyl oder -Alkenyl, Aryl, Heteroaryl, Cycloalkyl oder (Aryl, Heteroaryl oder Cycloalkyl)-niederalkyl, |

| | |
|---|---|
| L | H, nieder-Alkyl, Aryl, Cycloalkyl, oder (Aryl oder Cycloalkyl)-niederalkyl, oder |
| M und L | zusammen mit den Atomen, an die sie gebunden sind, eine Gruppe -N(Het) bilden, oder |
| E und G | zusammen eine Methylen- oder Carbonylgruppe bilden, und |
| M | H, nieder-Alkyl oder -Alkenyl, Aryl, Heteroaryl, Cycloalkyl oder (Aryl, Heteroaryl oder Cycloalkyl)-niederalkyl, oder Carboxy-niederalkyl, und |
| L | H, nieder-Alkyl, Aryl, Cycloalkyl, oder (Aryl oder Cycloalkyl)-niederalkyl, |
| A | H, Alkyl, nieder-Aralkyl oder eine Gruppe der Formel: |

$- C(O)R^2$ ($A^1$),

$-S(O)_2N(R^3,R^4)$ ($A^2$)

oder, falls die Gruppe Q eine Hydroxygruppe enthält, und/oder falls E und G zusammen $CH_2$ oder CO sind, dann

| | |
|---|---|
| A | auch eine Gruppe der Formel: |

$-S(O)_2R^5$ ($A^3$)

sein kann,

| | |
|---|---|
| $R^2$ | nieder-Alkyl, gegebenenfalls über nieder-Alkylen gebundenes Aryl, Heteroaryl oder Cycloalkyl, oder über nieder-Alkylen gebundenes Carbo-niederalkoxy oder über nieder-Alkylen gebundenes -(O oder S)-(Aryl, Heteroaryl oder Cycloalkyl), wobei eine in $R^2$ enthaltene nieder-Alkylengruppe in $\alpha$-Stellung zur Carbonylgruppe, an der $R^2$ gebunden ist, durch Hydroxy, Amino oder nieder-Alkanoylamino substituiert sein kann, oder |
| $R^2$ | über nieder-Alkylen gebundenes Halogen, Carboxy, nieder-Alkoxy, Amino, Mono- oder Di-niederalkyl-amino oder eine über nieder-Alkylen gebundene Gruppe -N(Het) oder |
| $R^2$ | eine Gruppe $-OR^{22}$ oder $-NHR^{22}$, |
| $R^{22}$ | nieder-Alkyl oder gegebenenfalls über nieder-Alkylen gebundenes Aryl, Heteroaryl oder Cycloalkyl, oder im nieder-Alkylanteil durch Aryl, Carbo-niederalkoxy oder COOH substituiertes nieder-Aralkyl, |
| $R^3$ und $R^4$ | unabhängig voneinander Wasserstoff, Alkyl oder Aryl-niederalkyl, oder zusammen mit dem N-Atom, an dem sie gebunden sind, eine Gruppe -N(Het) bilden, |
| $R^5$ | Aryl, Heteroaryl, Heterocyclyl, Alkyl oder Cycloalkyl, |
| -N(Het) | N-gebundenes, gegebenenfalls durch O, S, NH oder N-nieder-Alkyl unterbrochenes und gegebenenfalls durch bis zu 2 Substituenten aus der Gruppe von nieder-Alkyl, OH, Oxo, COOH, COO-nieder-Alkyl, $CH_2OH$ und $CH_2O$-Benzyl substituiertes $-N(CH_2)_{4-9}$, |
| Q | eine Gruppe der Formel $Q^1$ oder $Q^2$: |

($Q^1$) 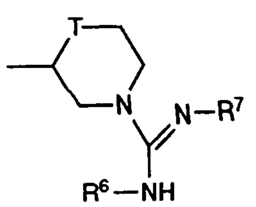 und ($Q^2$) 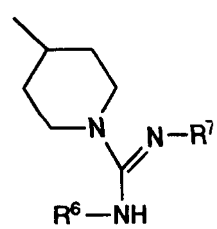

| | |
|---|---|
| T | $CH_2$ oder O, |
| eins von $R^6$ und $R^7$ | Wasserstoff oder Carbo-niederalkoxy und das andere Wasserstoff, Carbo-niederalkoxy oder Hydroxy, und |
| R | Wasserstoff oder nieder-Alkyl ist, |

sowie Hydrate oder Solvate und physiologisch verträgliche Salze davon.

**2.** Carboxamide nach Anspruch 1, worin

E Wasserstoff,

G H, nieder-Alkyl oder gegebenenfalls über nieder-Alkylen gebundenes COOH, COO-nieder-Alkyl, nieder-Alkanoyl, OH, nieder-Alkanoyloxy, nieder-Alkoxy, Aryl-niederalkoxy, $CONH_2$, $CONHCH_2CH_2OH$, CONHOH, $CONHOCH_3$, CONHO-Benzyl, $CONHSO_2$-nieder-Alkyl, $CONHCH_2CH_2$-Aryl, CONH-Cycloalkyl, $CONHCH_2$-Heteroaryl, $NH_2$, NHCOO-nieder-Alkyl, NHCOO-nieder-Aralkyl, $NHSO_3H$, ($NHSO_2$ oder $NHSO_3$)-nieder-Alkyl, NH-nieder-Alkanoyl, NHCOCOOH, NHCOCOO-nieder-Alkyl, NH-Cycloalkyl, NH-(3,4-Dioxo-2-hydroxy-cyclobut-1-enyl), NH-[2-nieder-(Alkoxy oder -Alkenyloxy)-3,4-dioxocyclobut-1-enyl], $NHCH_2$-Heteroaryl, NHCOCO-(Aryl oder nieder-Alkyl), $NHCOCH_2Cl$, $NHCOCH_2O$-Aryl, $NHCOCH_2$-Aryl, NHCO-(Aryl oder Heteroaryl), $NHPO_3$-($R^{10}$,$R^{20}$), Heteroaryl, oder gegebenenfalls durch O oder S unterbrochenes und gegebenenfalls durch bis zu 2 Substituenten aus der Gruppe von nieder-Alkyl, COOH, COO-nieder-Alkyl, $CH_2OH$ und $CH_2O$-Benzyl substituiertes $CON(CH_2)_{4-9}$, und

$R^{10}$ und $R^{20}$ H, nieder-Alkyl oder Phenyl,

M H, nieder-Alkyl oder -Alkenyl, Aryl, Heteroaryl, Cycloalkyl oder (Aryl, Heteroaryl oder Cycloalkyl)-niederalkyl,

L H, nieder-Alkyl, Aryl, Cycloalkyl, oder (Aryl oder Cycloalkyl)-niederalkyl, oder

M und L zusammen mit den Atomen, an die sie gebunden sind ein gegebenenfalls durch O oder S unterbrochenes und gegebenenfalls durch bis zu 2 Substituenten aus der Gruppe von nieder-Alkyl, COOH, COO-nieder-Alkyl, $CH_2OH$, $CH_2O$-Benzyl substituiertes $N(CH_2)_{4-9}$ bilden, oder

E und G bilden zusammen eine Methylen oder Carbonylgruppe, und

M H, nieder-Alkyl oder -Alkenyl, Aryl, Heteroaryl, Cycloalkyl oder (Aryl, Heteroaryl oder Cycloalkyl)-niederalkyl, oder Carboxy-niederalkyl, und

L H, nieder-Alkyl, Aryl, Cycloalkyl, oder (Aryl oder Cycloalkyl)-niederalkyl,

A eine Gruppe der Formel:

$-C(O)R^2$ $(A^1)$,

$-S(O)_2N(R^3,R^4)$ $(A^2)$

oder, falls die Gruppe Q eine Hydroxygruppe enthält,

A auch eine Gruppe der Formel

$-S(O)_2R^5$ $(A^3)$

sein kann oder, falls E und G zusammen $CH_2$ oder CO sind,

A auch H, Alkyl oder eine Gruppe der Formel:

$-S(O)_2R^5$ $(A^3)$

sein kann,

$R^2$ eine Gruppe $R^{22}$, $-OR^{22}$ oder $-NHR^{22}$, und

$R^{22}$ nieder-Alkyl oder gegebenenfalls über nieder-Alkylen gebundenes Aryl, Heteroaryl oder Cycloalkyl, oder

$R^2$ über nieder-Alkylen gebundenes Carbo-niederalkoxy oder über nieder-Alkylen gebundenes -(O oder S)-(Aryl, Heteroaryl oder Cycloalkyl), wobei eine in $R^2$ enthaltene nieder-Alkylengruppe in $\alpha$-Stellung zur Carbonylgruppe, an der $R^2$ gebunden ist, durch Hydroxy, Amino oder nieder-Alkanoylamino substituiert sein kann,

$R^3$ und $R^4$ unabhängig voneinander Wasserstoff, Alkyl oder Aryl-niederalkyl, oder zusammen mit dem N-Atom, an dem sie gebunden sind, gegebenenfalls durch O oder S unterbrochenes $-N(CH_2)_{4-9}$ sind,

$R^5$ Aryl, Heteroaryl, Heterocyclyl, Alkyl oder Cycloalkyl,

Q eine Gruppe der Formel $Q^1$ oder $Q^2$:

(Q1) 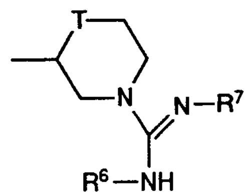 und (Q2) 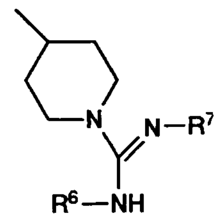

| | |
|---|---|
| T | $CH_2$ oder O, |
| eins von $R^6$ und $R^7$ | Wasserstoff oder Carbo-niederalkoxy und das andere Wasserstoff, Carbo-niederalkoxy oder Hydroxy, und |
| R | Wasserstoff ist, |

sowie Hydrate oder Solvate und physiologisch verträgliche Salze davon.

**3.** Carboxamide nach Anspruch 1 und der Formel

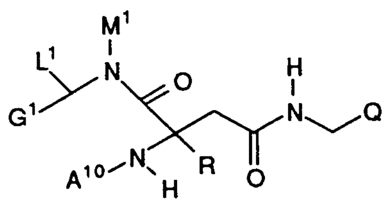

Ia

worin

| | |
|---|---|
| $G^1$ | H, nieder-Alkyl oder gegebenenfalls über nieder-Alkylen gebundenes COOH, COO-nieder-Alkyl, nieder-Alkanoyl, OH, nieder-Alkanoyloxy, nieder-Alkoxy, Aryl-niederalkoxy, $CONH_2$, $CONHCH_2CH_2OH$, CONHOH, $CONHOCH_3$, CONHO-Benzyl, $CONHSO_2$-nieder-Alkyl, $CONHCH_2CH_2$-Aryl, CONH-Cycloalkyl, $CONHCH_2$-Heteroaryl, $NH_2$, NHCOO-nieder-Alkyl, NHCOO-nieder-Aralkyl, $NHSO_3H$, ($NHSO_2$ oder $NHSO_3$)-nieder-Alkyl, NH-nieder-Alkanoyl, NHCOCOOH, NHCOCOO-nieder-Alkyl, NH-Cycloalkyl, NH-(3,4-Dioxo-2-hydroxycyclobut-1-enyl), NH-[2-nieder-(Alkoxy oder -Alkenyloxy)-3,4-dioxocyclobut-1-enyl], $NHCH_2$-Heteroaryl, NHCOCO-(Aryl oder nieder-Alkyl), $NHCOCH_2Cl$, NHCO-nieder-Alkylen-O-(niederalkyl oder aryl), $NHCOCH_2$[Aryl, Heteroaryl oder -N(Het)], NHCOC(NOH)-nieder-Alkylen-COOH, $NHSO_2$-N(Het), NHCO-(Aryl, Heteroaryl oder Heterocyclyl), $NHPO_3(R^{10},R^{20})$, Heteroaryl oder eine Gruppe CO-N(Het), |
| $R^{10}$ und $R^{20}$ | H, nieder-Alkyl oder Phenyl, und |
| $M^1$ | H, nieder-Alkyl oder -Alkenyl, Aryl, Heteroaryl, Cycloalkyl oder (Aryl, Heteroaryl oder Cycloalkyl)-niederalkyl, |
| $L^1$ | H, nieder-Alkyl, Aryl, Cycloalkyl, oder (Aryl oder Cycloalkyl)-niederalkyl, oder |
| $M^1$ und $L^1$ | zusammen mit den Atomen, an die sie gebunden sind eine Gruppe -N(Het) bilden, |
| $A^{10}$ | H, Alkyl, nieder-Aralkyl oder eine Gruppe der Formel: |

$-C(O)R^2$ ($A^1$),

$-S(O)_2N(R^3,R^4)$ ($A^2$)

oder, falls die Gruppe Q eine Hydroxygruppe enthält, dann

$A^{10}$ auch eine Gruppe der Formel

$-SO_2(R^5)$ ($A^3$)

sein kann, und

R, $R^2$ bis $R^5$, -N(Het) und Q die gleiche Bedeutung wie in Anspruch 1 haben,

sowie Hydrate oder Solvate und physiologisch verträgliche Salze davon.

**4.** Carboxamide nach Anspruch 1 und der Formel

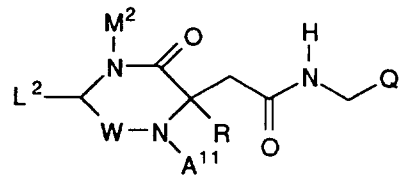

Ib

worin

| | |
|---|---|
| W | Methylen oder Carbonyl, |
| $M^2$ | H, nieder-Alkyl oder -Alkenyl, Aryl, Heteroaryl, Cycloalkyl oder (Aryl, Heteroaryl oder Cycloalkyl)-niederalkyl, oder Carboxy-niederalkyl, und |
| $L^2$ | H, nieder-Alkyl, Aryl, Cycloalkyl, oder (Aryl oder Cycloalkyl)-niederalkyl, und |
| $A^{11}$ | H, Alkyl, nieder-Aralkyl oder eine Gruppe der Formel: |

$-C(O)R^2$ ($A^1$),

$- S(O)_2N(R^3,R^4)$ ($A^2$)

oder

$-S(O)_2R^5$ ($A^3$)

ist, und

R, $R^2$ bis $R^5$ und Q die gleiche Bedeutung wie in Anspruch 1 haben,

sowie Hydrate oder Solvate und physiologisch verträgliche Salze davon.

**5.** Carboxamide nach Anspruch 3 worin

| | |
|---|---|
| $G^1$ | H, nieder-Alkyl oder gegebenenfalls über nieder-Alkylen gebundenes COOH, COO-nieder-Alkyl, nieder-Alkanoyl, OH, nieder-Alkanoyloxy, nieder-Alkoxy, Aryl-niederalkoxy, $CONH_2$, $CONHCH_2CH_2OH$, CONHOH, $CONHOCH_3$, CONHO-Benzyl, $CONHSO_2$-nieder-Alkyl, $CONHCH_2CH_2$-Aryl, CONH-Cycloalkyl, $CONHCH_2$-Heteroaryl, $NH_2$, NHCOO-nieder-Alkyl, NHCOO-nieder-Aralkyl, $NHSO_3H$, ($NHSO_2$ oder $NHSO_3$)-nieder-Alkyl, NH-nieder-Alkanoyl, NHCOCOOH, NHCOCOO-nieder-Alkyl, NH-Cycloalkyl, NH-(3,4-Dioxo-2-hydroxy-cyclobut-1-enyl), NH-[2-nieder-(Alkoxy oder -Alkenyloxy)-3,4-dioxocyclobut-1-enyl], $NHCH_2$-Heteroaryl, NHCOCO-(Aryl oder nieder-Alkyl), $NHCOCH_2Cl$, $NHCOCH_2O$-Aryl, $NHCOCH_2$-Aryl, NHCO-(Aryl oder Heteroaryl), $NHPO_3(R^{10},R^{20})$, Heteroaryl oder gegebenenfalls durch O oder S unterbrochenes und gegebenenfalls durch bis zu 2 Substituenten aus der Gruppe von nieder-Alkyl, COOH, COO-nieder-Alkyl, $CH_2OH$ und $CH_2O$-Benzyl substituiertes $CON(CH_2)_{4-9}$, und |
| $R^{10}$ und $R^{20}$ | H, nieder-Alkyl oder Phenyl, |
| $M^1$ | H, nieder-Alkyl oder -Alkenyl, Aryl, Heteroaryl, Cycloalkyl oder (Aryl, Heteroaryl oder Cycloalkyl)-niederalkyl, |
| $L^1$ | H, nieder-Alkyl, Aryl, Cycloalkyl, oder (Aryl oder Cycloalkyl)-niederalkyl, oder |
| $M^1$ und $L^1$ | zusammen mit den Atomen, an die sie gebunden sind ein gegebenenfalls durch O oder S unterbrochenes und gegebenenfalls durch bis zu 2 Substituenten aus der Gruppe von nieder-Alkyl, COOH, COO-nieder-Alkyl, $CH_2OH$, $CH_2O$-Benzyl substituiertes $N(CH_2)_{4-9}$ bilden, |
| $A^{10}$ | eine Gruppe der Formel: |

-C(O)$R^2$ ($A^1$),

-$S(O)_2N(R^3,R^4)$ ($A^2$)

$A^{10}$ oder, falls die Gruppe Q eine Hydroxygruppe enthält, auch eine Gruppe der Formel

- $SO_2(R^5)$ ($A^3$)

sein kann,

R Wasserstoff ist, und

$R^2$ bis $R^5$ und Q die gleiche Bedeutung wie in Anspruch 3 haben,

sowie Hydrate oder Solvate und physiologisch verträgliche Salze davon.

**6.** Carboxamide nach Anspruch 4 worin

R Wasserstoff,

$A^{11}$ H, Alkyl oder eine Gruppe der Formel:

-C(O)$R^2$ ($A^1$),

-$S(O)_2N(R^3,R^4)$ ($A^2$)

oder

-$S(O)_2R^5$ ($A^3$),

ist, und $L^2$, $M^2$, W, $R^2$ bis $R^5$ und Q die gleiche Bedeutung wie in Anspruch 4 haben, sowie Hydrate oder Solvate und physiologisch verträgliche Salze davon.

**7.** Carboxamide der Formel Ia nach Anspruch 3, worin $L^1$ Wasserstoff und $G^1$ gegebenenfalls über nieder-Alkylen gebundenes NHCO-nieder-Alkylen-O-(niederalkyl oder aryl), $NHCOCH_2$[Aryl, Heteroaryl oder -N(Het)], NHCOC(NOH)-nieder-Alkylen-COOH, $NHSO_2$-N(Het), NHCO-Heterocyclyl oder gegebenenfalls durch O oder S unterbrochenes und gegebenenfalls durch bis zu 2 Substituenton aus der Gruppe von nieder-Alkyl, COOH, COO-nieder-Alkyl, $CH_2OH$ und $CH_2O$-Benzyl substituiertes $CON(CH_2)_{4-9}$ ist.

**8.** Carboxamide der Formel Ia nach Anspruch 3, worin $L^1$ Wasserstoff und $G^1$ gegebenenfalls über nieder-Alkylen gebundenes COOH, COO-nieder-Alkyl, NHCOO-nieder-Aralkyl oder NHCO(Aryl oder Heteroaryl) ist.

**9.** Carboxamide der Formel Ia nach Anspruch 3, 7 oder 8, worin $M^1$ nieder-Alkyl oder Cycloalkyl ist.

**10.** Carboxamide der Formel Ia nach Anspruch 3 oder 7-9, worin $A^{10}$ H, nieder-Alkyl, nieder-Aralkyl oder eine Gruppe

-C(O)-$R^2$ ($A^1$)

in der $R^2$ über nieder-Alkylen gebundenes Halogen, Carboxy, nieder-Alkoxy, Amino, Mono- oder Diniederalkyl-amino oder eine über nieder-Alkylen gebundene Gruppe -N(Het) oder
$R^2$ eine Gruppe -$NHR^{22}$, worin $R^{22}$ eine im nieder-Alkylanteil durch Aryl, Carbo-niederalkoxy oder COOH substituierte nieder-Aralkylgruppe ist.

**11.** Carboxamide der Formel Ia nach Anspruch 3 oder 7-10, worin $A^{10}$ eine Gruppe der Formel

-C(O)-$R^2$ ($A^1$)

in der $R^2$ eine Gruppe $R^{22}$, -$OR^{22}$ oder -$NHR^{22}$ ist, und $R^{22}$ nieder-Alkyl oder gegebenenfalls über nieder-Alkylen gebundenes Aryl, Heteroaryl oder Cycloalkyl ist, oder in der $R^2$ über nieder-Alkylen gebundenes Carbo-niederalkoxy oder über nieder-Alkylen gebundenes -(O oder S)-(Aryl oder Heteroaryl) ist, wobei eine in $R^2$ enthaltene nieder-Alkylengruppe in $\alpha$-Stellung zur Carbonylgruppe, an der $R^2$ gebunden ist, durch OH oder nieder-Alkanoylamino substituiert sein kann.

**12.** Carboxamide der Formel Ia nach Anspruch 3, 8 oder 9, worin $A^{10}$ Morpholinosulfonyl ist.

**13.** Carboxamide der Formel Ia nach Anspruch 3, 8 oder 9, worin $A^{10}$ eine Gruppe $-S(O)_2$Aryl und Q eine Hydroxygruppe enthält.

**14.** Carboxamide der Formel Ib nach Anspruch 4, worin $L^2$ Wasserstoff und $M^2$ nieder-Alkyl, Cycloalkyl oder Carboxy-niederalkyl ist.

**15.** Carboxamide der Formel Ib nach Anspruch 4 oder 14, worin $A^{11}$ Wasserstoff, nieder-Alkylsulfonyl oder Arylsulfonyl ist.

**16.** Carboxamide nach einem der Ansprüche 1-15, worin Q eine Gruppe der Formel $Q^1$, in der eins von $R^6$ und $R^7$ Wasserstoff und das andere Wasserstoff oder Hydroxy ist.

**17.** Verbindungen nach Anspruch 1 aus der Gruppe der folgenden:
(S)-N4-[(S)-1-(Amino-imino-methyl)-piperidin-3-ylmethyl]-N1-cyclo-propyl-2-morpholin-4-ylacetylamino-N1-(2-pyrazin-2-ylcarbonylamino-ethyl)-succinamid,
3-[[(S)-3-[(S)-1-(Amino-imino-methyl)-piperidin-3-ylmethylcarbamoyl]-2-(3-phenyl-propylamino)-propionyl]-cyclopropyl-amino]-propionsäure-ethylester,
3-[[(S)-3-[(S)-1-(Amino-imino-methyl)-piperidin-3-ylmethylcarbamoyl]-2-(3-phenyl-propylamino)-propionyl]-cyclopropyl-amino]-propionsäure,
3-[[(S)-3-[(S)-1-(Amino-imino-methyl)-piperidin-3-ylmethylcarbamoyl]-2-morpholin-4-ylsulfonylamino-propionyl]-cyclopropyl-amino]-propionsäure.

**18.** Verbindungen nach Anspruch 2 aus der Gruppe der folgenden:
[[(S)-3-[4-(Amino-imino-methyl)-morpholin-2-ylmethylcarbamoyl]-2-benzyloxycarbonylamino-propionyl]-butyl-amino]-essigsäure,
[[(S)-3-[(S)-1-(Amino-imino-methyl)-piperidin-3-ylmethylcarbamoyl]-2-pentanoylamino-propionyl]-cyclopropyl-amino]-essigsäure,
(S)-N4-[(S)-1-(Amino-imino-methyl)-piperidin-3-ylmethyl]-N1-cyclopropyl-N1-(2-pyrazin-2-ylcarbonylamino-ethyl)-2-pyrimidin-2-ylsulfanylacetylamino-succinamid,
(S)-N4-[(S)-1-(Amino-hydroxyimino-methyl)-piperidin-3-ylmethyl]-N1-cyclopropyl-2-(naphthalen-2-ylsulfonylamino)-N1-[2-(pyrazin-2-ylcarbonyl-amino)-ethyl]-succinamid,
(S)-N4-[(S)-1-(Amino-imino-methyl)-piperidin-3-ylmethyl]-N1-cyclo-propyl-2-morpholin-4-ylsulfonylamino-N1-[2-(6-oxo-1,6-dihydro-pyridazin-3-ylcarbonylamino-ethyl]-succinamid und
N-[(S)-1-(Amino-imino-methyl)-piperidin-3-ylmethyl]-2[(S)-4-cyclo-propyl-1-naphthalen-2-ylsulfonyl-3,6-dioxo-piperazin-2-yl]-acetamid

**19.** Verbindungen nach einem der Ansprüche 1 bis 18 zur Verwendung als Heilmittel, insbesondere als Hemmer der durch Thrombin induzierten Plättchenaggregation und Gerinnung von Fibrinogen im Blutplasma.

**20.** Verfahren zur Herstellung der Verbindungen nach einem der Ansprüche 1 bis 18, dadurch gekennzeichnet, dass man
a) eine Säure der Formel

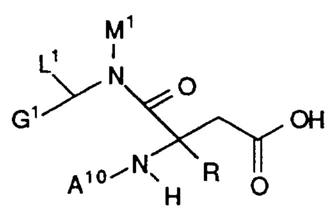

II

mit einem Amin der Formel

$H_2NCH_2Q$ III

oder einem Salz davon gegebenenfalls unter intermediärem Schutz von in den Gruppen $G^1$, $M^1$ und $A^{10}$ (in II) und Q (in III) enthaltenen funktionellen Gruppen umsetzt oder

b) ein Amin der Formel

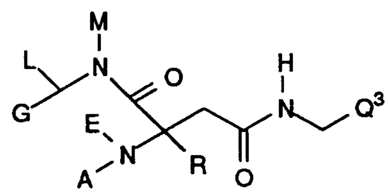

X

worin $Q^3$ eine Gruppe der Formel

($Q^{31}$) 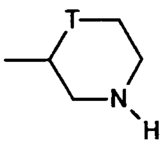 oder ($Q^{32}$) 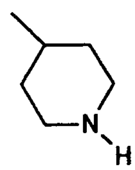

ist,

mit einem die gegebenenfalls hydroxylierte Amidingruppe -C($NR^7$)$NHR^6$ einführenden Mittel umsetzt oder

c) ein Amin der Formel

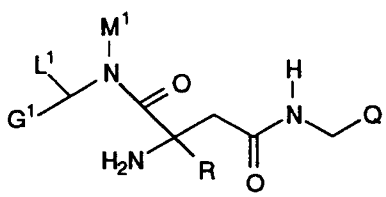

XX

mit einem die Gruppe $A^{10}$ einfuhrenden Mittel umsetzt oder

d) eine Aminosäure der Formel

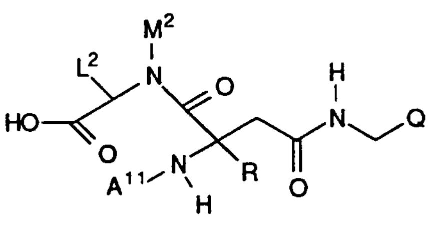

XXX

oder der Formel

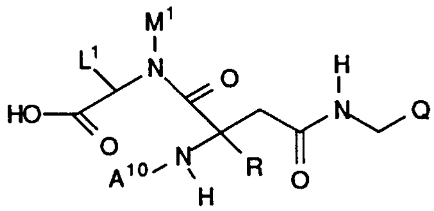

Ia1

zu einer Verbindung der Formel Ib cyclisiert oder

e) dass man zur Herstellung einer Carbonsäure der Formel Ib, worin $M^2$ Carboxy-niederalkyl ist, einen entsprechenden nieder-Alkylester spaltet und

f) gewünschtenfalls eine in einer Verbindung der Formel I enthaltene reaktionsfähige Gruppe funktionell abwandelt, und

g) gewünschtenfalls eine Verbindung der Formel I in ein physiologisch verträgliches Salz überführt oder ein Salz einer Verbindung der Formel I in die freie Säure oder Base überführt.

**21.** Pharmazeutische Präparate enthaltend eine Verbindung nach einem der Ansprüche 1 bis 18 als Wirkstoff.

**22.** Verwendung einer Verbindung nach einem der Ansprüche 1 bis 18 zur Herstellung von Arzneimitteln zur Behandlung oder Prophylase von Krankheiten, die durch Thrombin induzierter Plättchenaggregation oder Gerinnung von Fibrinogen im Blutplasma verursacht sind.

Europäisches Patentamt

# EUROPÄISCHER RECHERCHENBERICHT

Nummer der Anmeldung
EP 94 11 3488

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| P,X | EP-A-0 559 046 (HOFFMANN-LA ROCHE) 8. September 1993<br>* das ganze Dokument *<br>--- | 1-22 | C07D211/26<br>C07D265/30<br>A61K31/445<br>A61K31/535<br>C07D405/12<br>C07D401/12<br>C07D401/14 |
| X | EP-A-0 468 231 (HOFFMANN-LA ROCHE) 29. Januar 1992<br>* das ganze Dokument *<br>----- | 1-22 | |
| | | | RECHERCHIERTE SACHGEBIETE (Int.Cl.6) |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 13. Dezember 1994 | Kissler, B |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)